# FASCICULE DE BREVET EUROPEEN

(11) **EP 0 648 741 B1**
(45) Date de publication et mention de la délivrance du brevet: **18.12.1996**
(21) Numéro de dépôt: 94402299.5
(22) Date de dépôt: 13.10.1994
(51) Int. Cl.: C07C 311/20, C07D 311/58, C07D 335/06, C07D 213/55, A61K 31/18

(54) **Dérivés de 1,2,3,4-tétrahydronaphtalène, de chromanne et de thiochromanne comme agents antithromotiques**
1,2,3,4-Tetrahydronaphthalin-, Chroman- und Thiochroman-Derivate als Antithrombotika
1,2,3,4-Tetrahydronaphthalene, chroman and thiochroman derivatives as antithrombotic agents

(30) Priorité: 15.10.1993 FR 9312237
(43) Date de publication de la demande: 19.04.1995
(73) Titulaire: ADIR ET COMPAGNIE, F-92415 Courbevoie Cédex (FR)
(72) Inventeur: Lavielle, Gilbert, F-78170 La Celle Saint Cloud (FR); Dubuffet, Thierry, F-94240 l'Hay Les Roses (FR); Muller, Olivier, F-95300 Ennery (FR); Laubie, Michel, F-92420 Vaucresson (FR); Verbeuren, Tony, F-78540 Vernouillet (FR); Simonet, Serge, F-78700 Conflans Sainte Honorine (FR); Descombes, Jean-Jacques, F-93360 Neuilly-Plaisance (FR)

(56) Documents cités:
- EP-A- 0 135 177
- EP-A- 0 253 257
- EP-A- 0 430 459
- PATENT ABSTRACTS OF JAPAN vol. 17 no. 594 (C-1126) ,29 Octobre 1993 & JP-A-05 178814 (TANABE SEIYAKU) 20 Juillet 1993,

## Description

La présente invention concerne de nouveaux dérivés de 1,2,3,4-tétrahydronaphtalène, leur procédé de préparation et les compositions pharmaceutiques qui les contiennent.

Plus particulièrement, les composés décrits dans la présente invention possèdent des propriétés antithromboxane A₂ aussi bien en tant qu'antagonistes des récepteurs au thromboxane A₂ (TXA₂) qu'en tant qu'inhibiteurs de l'activité de l'enzyme responsable de la synthèse du thromboxane A₂ : la thromboxane A₂-synthase.

Le thromboxane A₂ est un métabolite de l'acide arachidonique produit par les plaquettes sanguines qui provoque une constriction importante des vaisseaux sanguins et induit l'agrégation des plaquettes. La production du thromboxane A₂ est augmentée dans des maladies comme l'angine de poitrine ou l'accident vasculaire cérébral et il joue un rôle très important dans tous les processus conduisant aux maladies thrombotiques.

Il était donc particulièrement intéressant de synthétiser des substances susceptibles d'inhiber les activités proagrégantes et vasoconstrictives du thromboxane A₂ soit en tant qu'antagonistes des récepteurs au thromboxane A₂, soit en tant qu'inhibiteur de la thromboxane A₂-synthase.

Les composés décrits dans la présente invention, outre le fait qu'ils soient nouveaux, possèdent des propriétés pharmacologiques nettement plus intenses que celles des autres composés décrits dans l'Art Antérieur.

Ils sont donc utiles en tant qu'antagonistes au thromboxane A₂ et en tant qu'inhibiteurs de la thromboxane A₂-synthase dans le traitement ou la prévention des maladies dans lesquelles le thromboxane A₂ est impliqué comme par exemple les maladies cardio et cérébrovasculaires et les maladies thrombotiques ainsi que les complications vasculaires qui accompagnent les conditions pathologiques impliquant soit le thromboxane A₂ ou des substances qui interagissent avec le récepteur TXA₂ (comme par exemple les complications vasculaires dans le diabète). Ces antagonistes au thromboxane A₂ possèdent également des propriétés protectrices de la paroi gastrique (M.L. OGLETREE et coll., J. Pharm. and Exp. Therap., 263 (1), 374-380). Enfin, leurs propriétés inhibitrices de l'agrégation plaquettaire leur permettent d'être également utiles dans le traitement de la migraine (P. PUIG-PARELLADA et coll., Prostaglandins Leukotrienes and Essential Fatty Acids, 49, 537-547, 1993).

Plus spécifiquement, la présente invention concerne les composés de formule (I) : 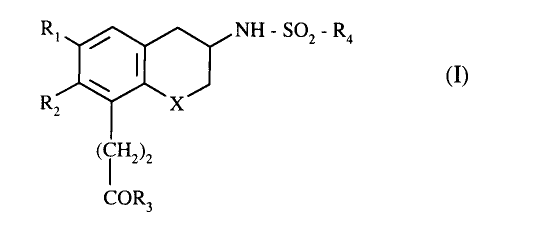 dans laquelle :
- R₁, R₂,: identiques ou différents, représentent un atome d'hydrogène, d'halogène, un groupement alkyle (C₁-C₆) linéaire ou ramifié, un groupement phényle (substitué ou non par un ou plusieurs atomes d'halogène ou groupements alkyl (C₁-C₆) linéaire ou ramifié, alkoxy (C₁-C₆) linéaire ou ramifié, hydroxy ou trihalogénométhyle), un groupement benzyle, un groupement pyridylméthyle, un groupement imidazolylméthyle, un groupement thiazolylméthyle, un groupement pyridyle, un groupement imidazolyle ou un groupement thiazolyle,
ou bien,
- R₁ et R₂: forment avec les atomes de carbone auxquels ils sont attachés un cycle cyclopentane ou cyclohexane,
- R₃: représente un groupement hydroxy, un groupement alkoxy (C₁-C₆) linéaire ou ramifié ou amino (substitué ou non par un ou deux groupements alkyle (C₁-C₆) linéaire ou ramifié),
- R₄: représente un groupement alkyle (C₁-C₆) linéaire ou ramifié, un groupement phényle (substitué ou non par un ou plusieurs atomes d'halogène ou groupements alkyle (C₁-C₆) linéaire ou ramifié, alkoxy (C₁-C₆) linéaire ou ramifié, trihalogénométhyle ou hydroxy), un groupement naphtyle, un groupement pyridyle, un groupement thiényle ou un groupement thiazolyle,
- X: représente un groupement méthylène, un atome d'oxygène ou de soufre,
leurs énantiomères, ainsi que leurs sels d'addition à une base pharmaceutiquement acceptable.

Parmi les bases pharmaceutiquement acceptables, on peut citer à titre non limitatif l'hydroxyde de sodium, l'hydroxyde de potassium, la tertbutylamine, la diéthylamine, l'éthylènediamine...

L'invention s'étend également au procédé de préparation des composés de formule (I).

Le procédé de préparation des composés de formule (I) dans laquelle X représente un groupement méthylène est caractérisé en ce que l'on utilise comme produit de départ un chlorure de sulfonyle de formule (II) :

R₄ - SO₂Cl (II)

dans laquelle R₄ a la même signification que dans la formule (I),
que l'on fait réagir en milieu basique :
- soit: sur une cyclohexylamine de formule (III) (préparée à partir de la cyclohexanone correspondante par réaction, sous atmosphère inerte, avec la benzylamine en présence de triacétoxyborohydrure de sodium puis hydrogénolyse) : 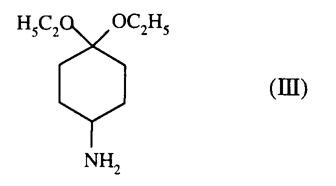 pour conduire au composé de formule (IV) : 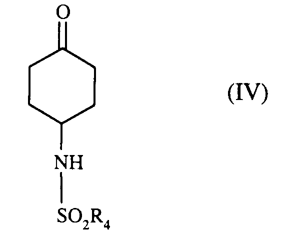 dans laquelle R₄ a la même signification que dans la formule (I),
- soit: sur le 4-aminocyclohexanol,
pour conduire au composé de formule (V) : 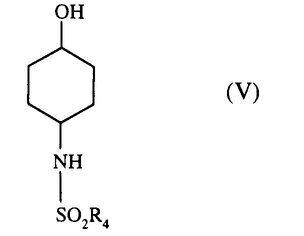 dans laquelle R₄ a la même signification que dans la formule (I),
que l'on oxyde à l'aide du réactif de Jones (acide chromique dans l'acétone et l'acide sulfurique aqueux),
pour conduire au composé de formule (IV) précédemment décrit,
composé de formule (IV),
que l'on fait réagir avec du formiate d'éthyle en présence d'hydrure de sodium,
pour conduire au composé de formule (VI) : 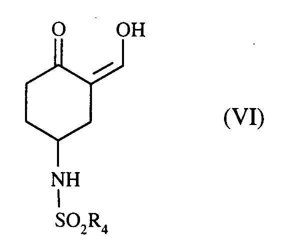
dans laquelle R₄ a la même signification que dans la formule (I),
qui subit ensuite, selon la nature des composés de formule (I) que l'on souhaite obtenir, l'action d'un carbalkoxyméthylènetriphénylphosphorane éventuellement substitué en α du groupe ester par un atome d'halogène, un groupement alkyle (C₁-C₆) linéaire ou ramifié, un groupement phényle (substitué ou non par un ou plusieurs atomes d'halogène ou groupement alkyl(C₁-C₆) linéaire ou ramifié, alkoxy (C₁-C₆) linéaire ou ramifié ou trihalogénométhyle), un groupement benzyle, un groupement pyridylméthyle ou un groupement imidazolylméthyle,
pour conduire au composé de formule (VII) : 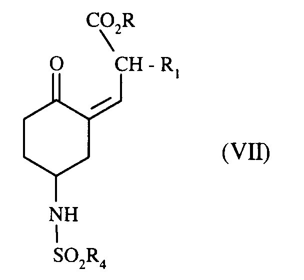
dans laquelle R₁ et R₄ ont la même signification que dans la formule (I) et R représente un groupement alkyle (C₁-C₆) linéaire ou ramifié,
que l'on met ensuite en réaction avec de l'acide p.toluènesulfonique ou l'acide trifluoroacétique,
pour conduire au composé de formule (VIII) : 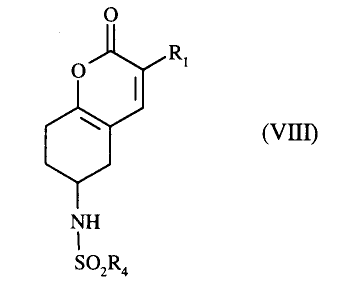
dans laquelle R₁ et R₄ ont la même signification que dans la formule (I),
qui subit une réaction de Diels-Alder avec un alc-2-ynoate d'alkyle convenablement substitué,
pour conduire, après transformations éventuelles, au composé de formule (IX) : 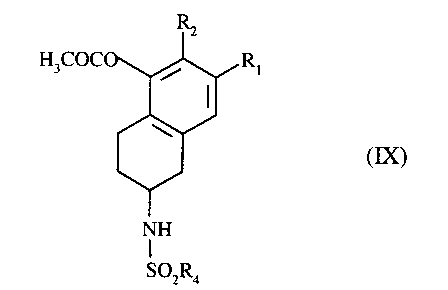
dans laquelle R₁, R₂ et R₄ ont la même signification que dans la formule (I),
qui subit l'action de l'hydrure de lithium aluminium en milieu anhydre,
pour conduire au composé de formule (X) : 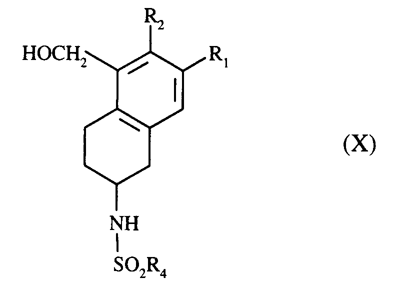
dans laquelle R₁, R₂ et R₄ ont la même signification que dans la formule (I),
que l'on fait réagir avec un agent d'oxydation comme le 4-benzylpyridinium dichromate,
pour conduire à l'aldéhyde de formule (XI) : 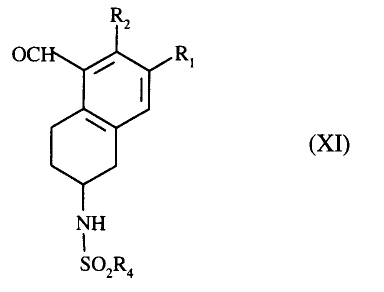
dans laquelle R₁, R₂ et R₄ ont la même signification que dans la formule (I),
que l'on fait réagir avec du (carbométhoxyméthylène)triphénylphosphorane,
pour conduire au composé de formule (XII) : 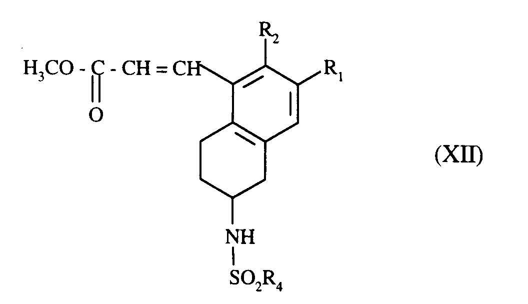 dans laquelle R₁, R₂ et R₄ ont la même signification que dans la formule (I),
que l'on réduit à l'aide d'iodure de samarium en présence de méthanol,
pour conduire au composé de formule (XIII) : 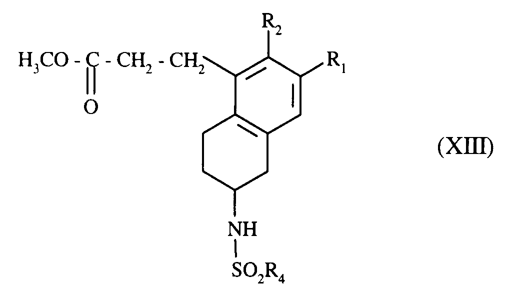 dans laquelle R₁, R₂ et R₄ ont la même signification que dans la formule (I),
que l'on transforme, ensuite en acide, ester ou amide correspondant, selon une technique classique de la chimie organique,
pour conduire au composé de formule (I/a), cas particulier des composés de formule (I) : 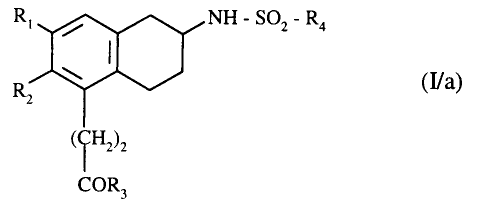 dans laquelle R₁, R₂, R₃ et R₄ ont la même signification que dans la formule (I),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

Le composé de formule (IX) décrit précédemment peut être obtenu :
a : soit directement par réaction du composé de formule (VIII) avec un alc-2-ynoate d'alkyle convenablement substitué de formule suivante :

   R₂ - C ≡ C - CO₂R

   dans laquelle :
   R représente un groupement alkyle (C₁-C₆) linéaire ou ramifié,
   et R₂ a la même signification que dans la formule (I),
b : soit, pour certaines valeurs du groupement R₂, par réaction avec un alc-2-ynoate d'alkyle de formule :

   R'₂ - C ≡ C - CO₂R

   dans laquelle R représente un groupement alkyle (C₁-C₆) linéaire ou ramifié,
   et R'₂ représente un groupement triméthylsilyle ou tributylstannanyle,
   pour conduire dans ce cas au composé de formule (IX') : 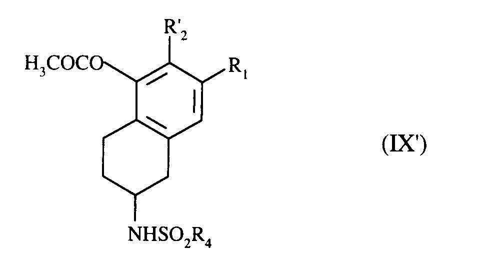 dans laquelle R₁, R₄ et R'₂ ont la même signification que précédemment, qui :
   - lorsque R'₂ représente un groupement triméthylsilyle est transformé :
      - soit en composé de formule (IX) dans laquelle R₂ représente un atome d'hydrogène,
      - soit en composé iodé correspondant qui, lui-même, subit l'action du tétraméthylétain en présence d'un catalyseur métallique, pour conduire au composé de formule (IX) dans laquelle R₂ représente un groupement méthyle,
   - lorsque R'₂ représente un groupement tributylstannanyle est transformé :
      - soit en composé de formule (IX) dans laquelle R₂ représente un atome de brome, dont on peut, si on le souhaite, substituer l'atome de brome par un substituant R₂ tel que défini dans la formule (I) en présence d'un catalyseur approprié,
      - soit directement en composé de formule (IX) dans laquelle R₂ représente un groupement alkyle (C₁-C₆) linéaire ou ramifié ou un groupement phényle substitué ou non.

Les composés de formule (I) dans lesquels R₁ représente un groupement benzyle, pyridylméthyle, thiazolylméthyle ou imidazolylméthyle et X représente un groupement méthylène, sont plus particulièrement obtenus à partir du composé de formulé (XI) décrit précédemment dans laquelle R₁ représente un atome de brome que l'on fait réagir avec le dérivé de formule suivante :

R₁ - Sn(C₄H₉)₃

conduisant au composé de formule (XI) convenablement substitué et subissant alors la suite de réactions décrites précédemment pour la transformation du composé de formule (XI) au composé de formule (I/a).

Lorsque les composés de formule (I) que l'on souhaite obtenir sont tels que R₁ et R₂ représentent chacun un atome d'hydrogène et X un groupement méthylène, ces composés peuvent être obtenus selon le procédé caractérisé en ce que l'on utilise comme produit de départ un composé de formule (XIV) : 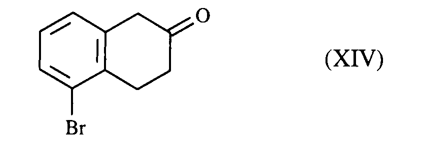 que l'on fait réagir avec un excès d'acrylate de méthyle en présence d'une quantité catalytique d'acétate de palladium et de triorthotolylphosphine dans la triéthylamine,
pour conduire au composé de formule (XV) : 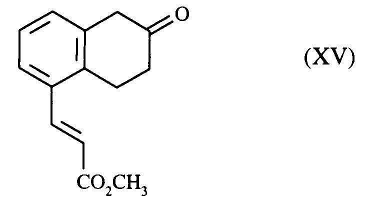 que l'on transforme en composé de formule (XVI) par réaction avec la benzylamine en présence de triacétoxyborohydrure de sodium, 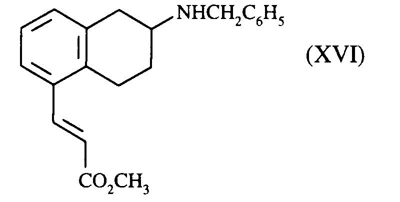 qui subit une hydrogénation catalytique pour conduire au composé de formule (XVII) : 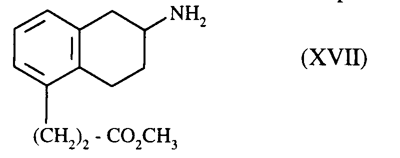 sur lequel on fait réagir un chlorure de sulfonyle de formule (II), en milieu basique :

R₄SO₂Cl (II)

dans laquelle R₄ a la même signification que dans la formule (I),
pour conduire au composé de formule (XVIII) : 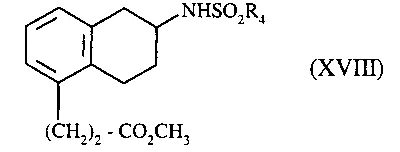 dans laquelle R₄ a la même signification que dans la formule (I),
que l'on transforme, ensuite en acide, ester ou amide correspondant, selon une technique classique de la chimie organique,
pour conduire au composé de formule (I/b), cas particulier des composés de formule (I) : 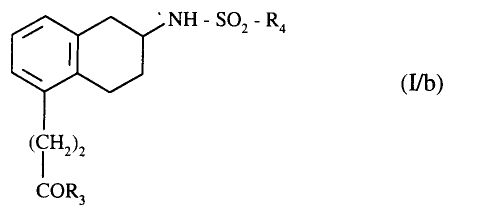 dans laquelle R₃ et R₄ ont la même signification que dans la formule (I),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

Le procédé de préparation des composés de formule (I) dans laquelle X (= X') représente un atome de soufre ou d'oxygène est caractérisé en ce que l'on utilise comme produit de départ un composé de formule (XIX) : 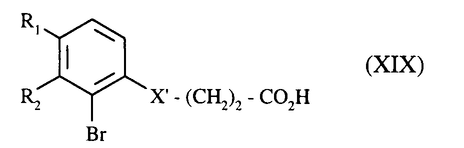 dans laquelle R₁ et R₂ ont la même signification que dans la formule (I) et X' représente un atome de soufre ou d'oxygène,
que l'on cyclise en présence d'acide polyphosphorique pour conduire au composé de formule (XX) : 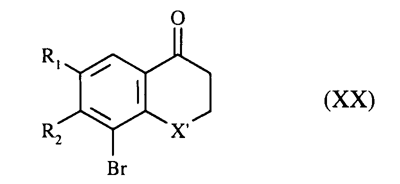 dans laquelle R₁, R₂ et X' ont la même signification que précédemment,
que l'on fait réagir avec un excès d'acrylate de méthyle en présence d'une quantité catalytique d'acétate de palladium et de triorthotolylphosphine dans la triéthylamine,
pour conduire au composé de formule (XXI) : 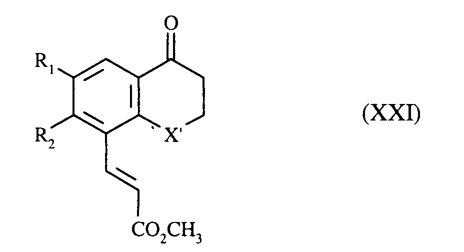 dans laquelle R₁, R₂ et X' ont la même signification que précédemment,
que l'on fait réagir avec de l'hydroxylamine, puis avec du chlorure de tosyle et enfin qui subit la transformation de Neber pour conduire au composé de formule (XXII) : 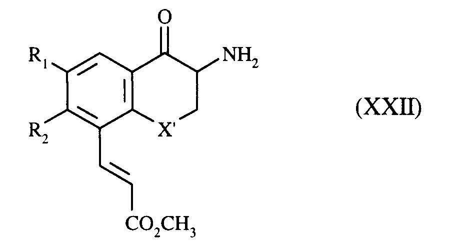 dans laquelle R₁, R₂ et X' ont la même signification que précédemment,
qui subit alors une réduction en présence d'un catalyseur, pour conduire au composé de formule (XXIII) : 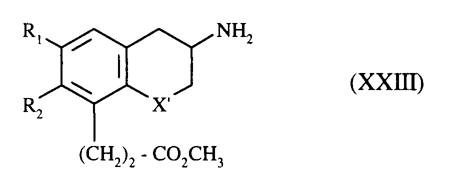 dans laquelle R₁, R₂ et X' ont la même signification que précédemment,
sur lequel on fait réagir un chlorure de sulfonyle de formule (II), en milieu basique :

R₄SO₂Cl (II)

dans laquelle R₄ a la même signification que dans la formule (I),
pour conduire au composé de formule (XXIV) : 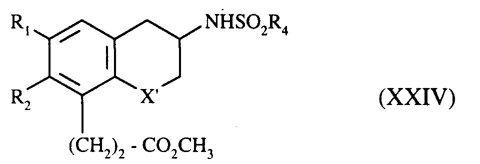 dans laquelle R₁, R₂, R₄ et X' ont la même signification que précédemment,
que l'on transforme, ensuite en acide, ester ou amide correspondant, selon une technique classique de la chimie organique,
pour conduire au composé de formule (I/c), cas particulier des composés de formule (I) : 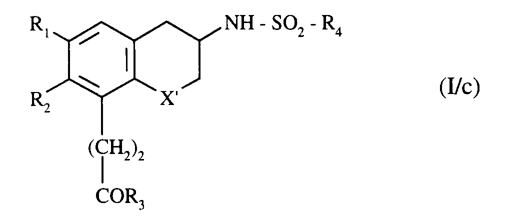 dans laquelle R₁, R₂, R₃, R₄ et X' ont la même signification que précédemment,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

Lorsque les composés de formule (I) que l'on souhaite obtenir sont tels que R₁ représente un groupement méthyle, R₂ un atome d'hydrogène et X représente un groupement méthylène, ces composés peuvent être plus particulièrement obtenus selon le procédé caractérisé en ce que l'on utilise comme produit de départ la 1,3-cyclohexanedione que l'on fait réagir avec l'aldéhyde tiglique pour conduire au composé de formule (XXV) : 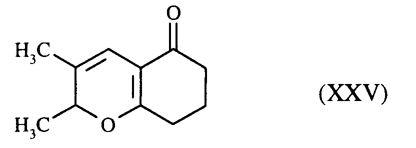 que l'on fait réagir avec du propiolate d'alkyle pour conduire au composé de formule (XXVI) : 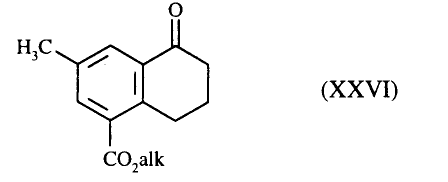 dans laquelle alk représente un groupement alkyle, que l'on fait réagir avec de l'hydroxylamine, puis avec du chlorure de tosyle et enfin qui subit une transformation de Neber,
pour conduire au composé de formule (XXVII) : 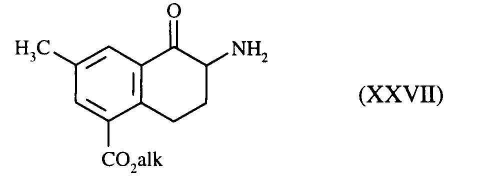 dans laquelle alk a la même signification que précédemment, qui subit alors une réduction catalytique, puis l'action du chlorure du sulfonyle de formule (II) décrit précédemment,
pour conduire au composé de formule (XXVIII) : 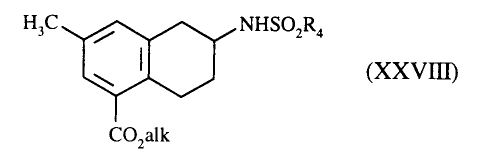 dans laquelle R₄ et alk ont la même signification que précédemment,
composé de formule (XXVIII) qui subit alors la suite de réactions décrite précédemment pour la transformation du composé de formule (IX) en composé de formule (I/a) et qui conduit au composé de formule (I) correspondant.

Les isomères des composés de formule (I) peuvent être obtenus par une technique classique de séparation à la fin de la synthèse ou à toute étape de la synthèse permettant une telle séparation.

Les composés de formule (I) possèdent des propriétés pharmacologiques très intéressantes. En particulier, ils sont capables d'inhiber l'agrégation plaquétaire induite par le U46619 (9,11-didéoxy-11α9α-époxyméthanoprostaglandine F₂α), agoniste des récepteurs TXA₂, d'inhiber les contractions provoquées par le U46619 sur la trachée de cobaye et de prévenir in vivo les bronchoconstrictions induites par le U46619 chez le cobaye. En plus, les composés inhibent la synthèse de TXA₂ dans le sang du lapin. Les composés de l'invention possèdent des activités pharmacologiques nettement plus intenses que celles d'un composé de référence, le BAY U3405 (Drug of the Future, 16(8), 701-705, 1991).

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 10 et 200 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus. Les structures chimiques des composés décrits dans les exemples ont été déterminées selon les techniques spectroscopiques habituelles (résonance magnétique nucléaire du Proton et du carbone 13, spectre de masse...)

### EXEMPLE 1 : Acide 3-{6-[(4-chlorophénylsulfonyl)amino]-2-méthyl-5,6,7,8-tétrahydronapht-1-yl}propionique, sel de sodium

### Stade A : 4,4-Diéthoxy-N-benzylcyclohexylamine

Ce composé est obtenu selon le procédé décrit dans Tet. Lett., 5595-5598, 1990. A 270 mmoles de 4,4-diéthoxycyclohexanone dans 700 ml de 1,2-dichloroéthane anhydre agitées, à température ambiante, sous atmosphère d'azote, sont ajoutés 270 mmoles de benzylamine dans 50 ml de 1,2-dichloroéthane, 270 mmoles d'acide acétique puis 80 g de triacétoxyborohydrure de sodium. Après 3 heures d'agitation, 1 l d'une solution saturée d'hydrogénocarbonate de sodium est versé sur le milieu réactionnel. Le pH est amené à 8 à l'aide de soude 1N. La phase organique est récupérée, séchée et évaporée. Le produit attendu est alors obtenu après purification de l'huile résiduelle par chromatographie sur colonne de silice en utilisant comme éluant un mélange dichlorométhane/ méthanol/ammoniaque (95/5/0,5).
- Rendement :: 80 %

### Stade B : 4,4-Diéthoxycyclohexylamine, oxalate

A 200 mmoles du composé obtenu au stade précédent dans 1,5 l d'éthanol anhydre, sont ajoutées, goutte à goutte, 200 mmoles d'acide oxalique diluées dans de l'éthanol anhydre, puis 6,1 g de palladium sur charbon. Le milieu réactionnel est hydrogéné 5 heures à 45°C sous pression d'hydrogène. Après filtration du catalyseur, le produit attendu est obtenu sous forme solide après évaporation.
- Rendement :: 90 %

| Microanalyse élémentaire : | | | |
|---|---|---|---|
| | C % | H % | N % |
| calculé | 51,97 | 8,36 | 5,05 |
| trouvé | 51,58 | 8,21 | 5,28 |

### Stade C : 4-(4-Chlorophénylsulfonyl)amino cyclohexanone

A une solution contenant 36 mmoles de chlorure de 4-chlorophénylsulfonyle dans 100 ml de tétrahydrofurane (THF) sont ajoutées 18 mmoles du composé obtenu au stade précédent dans 100 ml d'eau ainsi qu'une solution d'hydroxyde de potassium 1N de façon à maintenir le pH à 9. Après 90 minutes d'agitation, le milieu réactionnel est traité par de l'acide chlorhydrique 1N jusqu'à pH 3, dilué avec 50 ml d'eau puis extrait à l'éther. La phase étherée est alors séchée et évaporée. Le produit attendu est alors obtenu par purification du solide résiduel par chromatographie sur colonne de silice en utilisant comme éluant un mélange cyclohexane/acétate d'éthyle (70/30).
- Rendement :: 82 %

### Stade D : 4-(4-Chlorophénylsulfonyl)amino-2-hydroxyméthylène cyclohexanone

Ce composé est obtenu selon le procédé décrit dans Synthesis, 796-797, 1983. A 78 mmoles d'hydrure de sodium (lavées au pentane anhydre) diluées dans du THF anhydre, sont ajoutées, goutte à goutte, sous azote, 195 mmoles de formiate d'éthyle dans 25 ml de THF, puis, goutte à goutte, 19 mmoles du composé obtenu au stade précédent dans 40 ml de THF. L'agitation est maintenue 90 minutes. Après dilution du milieu par de l'eau, addition d'acide chlorhydrique jusqu'à pH 4, le produit attendu est extrait à l'éther et est obtenu après séchage et évaporation de la phase étherée.

### Stade E : 3-[5-(4-Chlorophénylsulfonyl)amino-2-oxo-cyclohexylidène]propanoate de méthyle

20 mmoles du composé obtenu au stade précédent et 23 mmoles de (carbométhoxyméthylidène)triphénylphosphorane sont mélangées dans 200 ml de chloroforme anhydre. L'ensemble est porté deux heures à reflux. Après refroidissement et évaporation du solvant, on obtient le produit attendu.

### Stade F : 6-(4-Chlorophénylsulfonyl)amino-2-oxo-5,6,7,8-tétrahydro-2H-benzo[e]pyrane

Ce composé est obtenu selon le procédé décrit dans Tetrahedron, 24(7), 2851-2858, 1968. 22 mmoles d'acide p.toluènesulfonique dissoutes dans 315 ml de toluène anhydre sont placées dans un tricol équipé d'un Dean-Stark et l'ensemble est porté à reflux 45 minutes. Le Dean-Stark est alors retiré et 20 mmoles du composé obtenu au stade précédent dans 150 ml de toluène anhydre sont ajoutées au mélange qui est porté 4 heures à reflux. Le milieu est alors dilué à l'eau. Après extraction à l'éther, séchage et évaporation, le produit attendu est obtenu sous forme solide après purification du résidu par chromatographie sur colonne de silice en utilisant comme éluant un mélange cyclohexane/acétate d'éthyle (50/50).
- Point de fusion :: 215°C

### Stade G : 6-(4-Chlorophénylsulfonyl)amino-2-méthyl-5,6,7,8-tétrahydronapht-1-yl-carboxylate de méthyle

22 mmoles du composé obtenu au stade précédent sont placées dans un autoclave avec 12,4 ml de but-2-ynoate de méthyle et l'ensemble est porté 48 heures à 170°C. Le produit attendu est alors obtenu après purification par chromatographie sur colonne de silice en utilisant comme éluant un mélange cyclohexane/acétate d'éthyle (80/20).

### Stade H : 6-(4-Chlorophénylsulfonyl)amino-2-méthyl-1-hydroxyméthyl-5,6,7,8-tétrahydronaphtalène

19 mmoles d'hydrure de lithium aluminium sont placées sous agitation dans 50 ml de THF anhydre. 9,5 mmoles du composé décrit au stade précédent dans 20 ml de THF sont alors ajoutées et l'ensemble est agité une heure. Après addition de 5 ml de méthanol puis de 20 ml d'eau, le produit attendu est obtenu après extraction à l'éther, séchage et évaporation.

### Stade I : 6-(4-Chlorophénylsulfonyl)amino-2-méthyl-5,6,7,8-tétrahydronapht-1-yl-carboxaldéhyde

Ce composé est obtenu selon le procédé décrit dans Synthetic Communications, 21(3), 419-425, 1991. 440 mmoles de 4-benzylpyridinium dichromate sont placées sous agitation dans 225 ml de dichlorométhane. 7 mmoles du composé obtenu au stade précédent dans 10 ml de dichlorométhane sont alors ajoutées et l'ensemble est agité une heure à température ambiante. 200 ml d'un mélange éther/hexane (1/1) sont additionnés à l'ensemble. Le précipité formé est filtré. Les filtrats sont récupérés, évaporés et le produit attendu est obtenu après purification du résidu par chromatographie sur colonne de silice en utilisant comme éluant un mélange cyclohexane/acétate d'éthyle (80/20).

### Stade J : 3-[6-(4-Chlorophénylsulfonyl)amino-2-méthyl-5,6,7,8-tétrahydronapht-1-yl]prop-2-ènoate de méthyle

4 mmoles du composé obtenu au stade précédent sont portées 48 heures à reflux dans 120 ml de chloroforme en présence de 5,2 mmoles de (carbométhoxyméthylidène) triphénylphosphorane. Les solvants sont évaporés et le produit attendu est obtenu après purification du résidu par chromatographie sur colonne de silice en utilisant come éluant un mélange cyclohexane/acétate d'éthyle (60/40).

### Stade K : 3-[6-(4-Chlorophénylsulfonyl)amino-2-méthyl-5,6,7,8-tétrahydronapht-1-yl]propanoate de méthyle

Ce composé est obtenu selon le procédé décrit dans J.A.C.S., 2693-2698, 1980. A 165 ml d'une solution 0,1N d'iodure de samarium dans le THF, placés sous agitation, sous atmosphère d'argon, est ajoutée 1,1 mmoles du composé obtenu au stade précédent. L'ensemble est agité 30 minutes à température ambiante. Après addition de 0,5 ml de méthanol, l'ensemble est agité 30 minutes supplémentaires. Après traitement par 200 ml d'une solution d'acide chlorhydrique 0,1N et extraction à l'éther, les phases organiques réunies sont lavées à l'eau, puis par une solution saturée de thiosulfate de sodium et à nouveau à l'eau. Après séchage et évaporation, on obtient le produit attendu.

### Stade L : Acide 3-{6-[(4-chlorophénylsulfonyl)amino]-2-méthyl-5,6,7,8-tétrahydronapht-1-yl}propionique, sel de sodium

1 mmole du composé obtenu au stade précédent est portée à reflux dans 10 ml de méthanol en présence de 3 équivalents de soude 2N, pendant une heure. Après refroidissement, l'ensemble est acidifié avec de l'acide chlorhydrique 1N et après extraction à l'acétate d'éthyle, séchage et évaporation, le résidu est repris par 5 ml de méthanol et est traité par un équivalent de soude 1N. Le produit attendu est alors obtenu après évaporation du solvant. La pureté est vérifiée par CLHP et le spectre de résonance magnétique nucléaire de proton réalisé dans le DMSO d₆ en présence de TMS confirme la structure du produit.

### EXEMPLE 2 : Acide 3-{6-[(4-chlorophénylsulfonyl)amino]-2,3-diméthyl-5,6,7,8-tétrahydronapht-1-yl}propionique, sel de sodium

### Stades A à D : Ces stades sont identiques aux stades A à D de l'exemple 1.

### Stade E : 2-Méthyl-3-[5-(4-chlorophénylsulfonyl)amino-2-oxo-cyclohexylidène]propanoate de méthyle

Le produit attendu est obtenu selon le procédé décrit au stade E de l'exemple 1 en remplaçant le (carbométhoxyméthylidène)triphénylphosphorane par le (carbéthoxyéthylidène) triphénylphosphorane.

### Stade F : 6-(4-Chlorophénylsulfonyl)amino-3-méthyl-2-oxo-4a,5,6,7,8,8a-hexahydro-2H-benzo[e]pyrane

Le produit attendu est obtenu en procédant comme au stade F de l'exemple 1 à partir du composé obtenu au stade précédent.

### Stade G : 6-(4-Chlorophénylsulfonyl)amino-2,3-diméthyl-5,6,7,8-tétrahydronapht-1-yl-carboxylate de méthyle

Le mode opératoire est identique à celui décrit au stade G de l'exemple 1.

### Stade H : 6-(4-Chlorophénylsulfonyl)amino-2,3-diméthyl-1-hydroxyméthyl-5,6,7,8-tétrahydronaphtalène

Le mode opératoire est identique à celui décrit au stade H de l'exemple 1.

### Stade I : 6-(4-Chlorophénylsulfonyl)amino-2,3-diméthyl-5,6,7,8-tétrahydronapht-1-yl-carboxaldéhyde

Le mode opératoire est identique à celui décrit au stade I de l'exemple 1.

### Stade J : 3-[6-(4-Chlorophénylsulfonyl)amino-2,3-diméthyl-5,6,7,8-tétrahydronapht-1-yl]prop-2-ènoate de méthyle

Le mode opératoire est identique à celui décrit au stade J de l'exemple 1.

### Stade K : 3-[6-(4-Chlorophénylsulfonyl)amino-2,3-diméthyl-5,6,7,8-tétrahydronapht-1-yl]propanoate de méthyle

Le mode opératoire est identique à celui décrit au stade K de l'exemple 1.

### Stade L : Acide 3-{6-[(4-chlorophénylsulfonyl)amino]-2,3-diméthyl-5,6,7,8-tétrahydronapht-1-yl}propionique, sel de sodium

Le mode opératoire est identique à celui décrit au stade L de l'exemple 1.La pureté du produit attendu est vérifiée par CLHP et le spectre de résonance magnétique nucléaire (RMN) de proton réalisé dans le DMSO en présence de TMS confirme la structure du produit.

### EXEMPLE 3 : Acide 3-{6-[(4-fluorophénylsulfonyl)amino]-2-méthyl-5,6,7,8-tétrahydronapht-1-yl}propionique, sel de sodium

Le produit attendu est obtenu selon le procédé décrit dans l'exemple 1 en remplaçant au stade C le chlorure de 4-chlorophénylsulfonyle par le chlorure de 4-fluorophénylsulfonyle. La pureté et la structure du produit attendu sont vérifiées par CLHP et RMN.

| Microanalyse élémentaire : | | | | |
|---|---|---|---|---|
| | C % | H % | N % | S % |
| calculé | 58,10 | 5,12 | 3,39 | 7,76 |
| trouvé | 58,12 | 5,62 | 3,54 | 7,91 |

### EXEMPLE 4 : Acide 3-{6-[(4-chlorophénylsulfonyl)amino]-2-propyl-5,6,7,8-tétrahydronapht-1-yl}propionique

Le produit attendu est obtenu sous forme d'acide libre selon le procédé décrit dans l'exemple 1 en remplaçant au stade G le but-2-ynoate de méthyle par l'hex-2-ynoate de méthyle. La pureté et la structure du produit attendu sont vérifiées par CLHP et RMN.
- Point de fusion :: 165-167°C

| Microanalyse élémentaire : | | | | | |
|---|---|---|---|---|---|
| | C % | H % | N % | Cl % | S % |
| calculé | 60,61 | 6,01 | 3,21 | 8,13 | 7,35 |
| trouvé | 61,03 | 6,27 | 3,24 | 8,54 | 7,19 |

### EXEMPLE 5 : Acide 3-{6-[(4-chlorophénylsulfonyl)amino]-3-chloro-2-méthyl-5,6,7,8-tétrahydronapht-1-yl}propionique, sel de sodium

### Stade A : 4-(4-Chlorophénylsulfonyl)aminocyclohexanol

A 100 g de chlorhydrate de trans 4-aminocyclohexanol en suspension dans 1 l de chloroforme, sont additionnées, à 5°C, 184 ml de triéthylamine puis une solution contenant 143 g de chlorure de 4-chlorophénylsulfonyl dans 150 ml de chloroforme. Après 2 heures de réaction, le mélange réactionnel est versé sur 500 ml d'eau. Le produit attendu est obtenu par filtration du précipité.
- Rendement :: 95 %
- Point de fusion :: 138-142°C

### Stade B : 4-(4-Chlorophénylsulfonyl)aminocyclohexanone

A 182 g du composé obtenu au stade précédent dissous dans 1,2 l d'acétone, sont ajoutés, sous vive agitation, à une température ne dépassant pas 35°C, 150 ml d'une solution oxydante obtenue en dissolvant 80 g d'anhydride chromique dans 190 ml d'eau et 70 ml d'acide sulfurique pur. Après une heure d'agitation, le précipité formé est filtré, rinçé à l'acétone. Les filtrats sont dilués par 250 ml d'alcool isopropylique et le pH de la solution amené à 7 à l'aide d'hydrogénocarbonate de sodium. Après filtration, évaporation des solvants, le résidu est repris par du dichlorométhane. La phase organique est lavée par de l'acide chlorhydrique 1N, de la soude 1N puis à l'eau. Après séchage et évaporation, on obtient le produit attendu.
- Rendement :: 70 %
- Point de fusion :: 103-105°C

### Stades C à K :

Les stades C à K sont identiques aux stades D à L de l'exemple 1 mais au stade D, le (carbométhoxyméthylène)triphénylphosphorane est remplacé par le (carbométhoxychlorométhylène)triphénylphosphorane. La pureté et la structure du produit attendu sont vérifiées par CLHP et RMN.

### EXEMPLE 6 : Acide 3-{6-[(4-chlorophénylsulfonyl)amino]-5,6,7,8-tétrahydronapht-1-yl}propionique, sel de sodium

### Stade A : 5-Bromo-2-oxo-1,2,3,4-tétrahydronaphtalène

1,12 mmoles de chlorure d'aluminium sont placées dans 1,5 l de chlorure de méthylène anhydre. A une température comprise entre -5°C et -10°C sont ajoutées 279 mmoles de chlorure d'acide phénylacétique dans 100 ml de dichlorométhane. Après 1 heure d'agitation, un bullage d'éthylène est effectué à cette température pendant 2 heures 30 minutes. On ajoute alors lentememt 1 l d'eau. La phase organique est décantée, séchée et évaporée. Le produit attendu est alors obtenu après purification par chromatographie sur colonne de silice en utilisant comme éluant un mélange cyclohexane/éther (95/5).
- Point de fusion :: 62°C

### Stade B : 3-(6-Oxo-5,6,7,8-tétrahydronapht-1-yl)prop-2-ènoate de méthyle

31 mmoles du composé obtenu au stade précédent sont placées dans 15 ml de triéthylamine en présence de 38 mmoles d'acrylate de méthyle, de 70 mg de diacétate de palladium et 370 mg de triorthotolylphosphine. L'ensemble est porté à 100°C pendant 10 heures en autoclave. Après addition de 300 ml d'acide chlorhydrique 1N, extraction au dichlorométhane, séchage et évaporation, le produit attendu est obtenu après purification du résidu par chromatographie sur colonne de silice en utilisant comme éluant un mélange cyclohexane/acétate d'éthyle (70/30).

### Stade C : 3-(6-Benzylamino-5,6,7,8-tétrahydronapht-1-yl)prop-2-ènoate de méthyle

14,7 mmoles du composé obtenu au stade précédent sont mises en solution dans 45 ml de 1,2-dichloroéthane. On additionne successivement 14,7 mmoles de benzylamine en solution dans 3 ml de 1,2-dichloroéthane puis 58,8 mmoles d'acide acétique. Après avoir agité le milieu réactionnel pendant 45 minutes, 82 mmoles de triacétoxyborohydrure de sodium sont ajoutées. Après 15 h, le milieu réactionnel est traité par une solution saturée de hydrogénocarbonate de sodium puis par de la soude 1N jusqu'à pH 8. Après décantation et évaporation des solvants, le produit brut est purifié par chromatographie en utilisant comme éluant un mélange cyclohexane/acétate d'éthyle (60/40).
- Rendement :: 86 %

### Stade D : 3-[6-Amino-5,6,7,8-tétrahydronapht-1-yl]propanoate de méthyle, chlorhydrate

12,6 mmoles du composé obtenu au stade précédent sont mises en solution dans l'éthanol. On additionne ensuite 12,6 mmoles d'acide chlorhydrique en solution dans l'éthanol puis 200 mg de palladium sur charbon. Le milieu est porté à 50°C puis soumis à l'action de l'hydrogène. Après 48 h, le palladium est filtré puis les solvants sont évaporés.

### Stade E : 3-[6-(4-Chlorophénylsulfonyl)amino-5,6,7,8-tétrahydronapht-1-yl]propanoate de méthyle

Le composé obtenu au stade précédent est soumis à une réaction de sulfonylation selon le procédé décrit au stade A de l'exemple 5. Le produit est purifié par chromatographie sur colonne de silice en utilisant comme éluant un mélange cyclohexane/acétate d'éthyle (80/20).

### Stade F : Acide 3-{6-[(4-chlorophénylsulfonyl)amino]-5,6,7,8-tétrahydronapht-1-yl}propionique, sel de sodium

Le produit attendu est obtenu selon le procédé décrit au stade L de l'exemple 1.

| Microanalyse élémentaire : | | | | | |
|---|---|---|---|---|---|
| | C % | H % | N % | Cl % | S % |
| calculé | 54,88 | 4,61 | 3,37 | 8,52 | 7,71 |
| trouvé | 55,19 | 4,76 | 3,54 | 8,66 | 7,08 |

### EXEMPLE 7 : Acide 3-{6-[(4-chlorophénylsulfonyl)amino]-2-méthyl-5,6,7,8-tétrahydronapht-1-yl}propionique

Le composé de l'exemple 1sous forme d'acide libre peut également être obtenu selon le procédé suivant :

### Stades A et B : Ces stades sont identiques aux stades A et B de l'exemple 5.

### Stades C, D et E : Ces stades sont identiques aux stades D, E et F de l'exemple 1.

### Stade F : [6-(4-Chlorophénylsulfonyl)amino-2-triméthylsilyl-5,6,7,8-tétrahydronapht-1-yl]carboxylate d'éthyle

69 mmoles du composé obtenu au stade précédent et 276 mmoles de triméthylsilylpropynoate d'éthyle sont chauffées à 180°C en présence de 150 ml de décaline pendant 72 h. Après refroidissement et évaporation de la décaline, le produit brut est chromatographié sur colonne de silice en utilisant comme éluant un mélange cyclohexane/acétate d'éthyle (80/20).
- Rendement :: 72 %

### Stade G : [6-(4-Chlorophénylsulfonyl)amino-2-iodo-5,6,7,8-tétrahydronapht-1-yl]carboxylate d'éthyle

Le produit attendu est obtenu selon le procédé décrit dans Angew. Chem. Int., 7, 488-489, 1977. 46 mmoles du composé obtenu au stade précédent en solution dans 300 ml de dichlorométhane sontmis en présence de 46 mmoles d'ICl (solution 1M dans le dichlorométhane). Après 1 h 30, les solvants sont évaporés et le produit brut est chromatographié sur colonne de silice en utilisant comme éluant un mélange cyclohexane/acétate d'éthyle (80/20).
- Rendement :: 85 %

### Stade H : 6-(4-Chlorophénylsulfonyl)amino-2-méthyl-5,6,7,8-tétrahydronapht-1-yl]carboxylate d'éthyle

Le produit attendu est obtenu selon le procédé décrit dans Tet. Lett., 33, 17, 2413-2416, 1992. A 39 mmoles du composé obtenu au stade précédent dans 200 ml de N-méthyl pyrrolidone, on ajoute successivement 196 mmoles de tétraméthylétain puis 2 mmoles de tétrakis triphénylphosphine palladium. Le milieu réactionnel est porté à 110°C pendant 8 h. Les solvants sont ensuite évaporés et le produit brut est chromatographié sur colonne de silice en utilisant comme éluant un mélange cyclohexane/acétate d'éthyle (80/20).
- Rendement :: 95 %

### Stades I, J, K et L : Ces stades sont identiques aux stades H, I, J, K de l'exemple 1.

### Stade M : Acide 3-[6-(4-chlorophénylsulfonyl)amino-2-méthyl-5,6,7,8-tétrahydronapht-1-yl]propionique

30 mmoles du produit obtenu au stade précédent sont mis en solution dans 10 ml de méthanol en présence de 90 mmoles d'une solution aqueuse 1N de soude. Le milieu réactionnel est porté 30 minutes à reflux puis acidifié par de l'acide chlorhydrique 1N. Le produit attendu est alors extrait à l'acétate d'éthyle et les solvants sont évaporés.
- Point de fusion :: 203°C

| Microanalyse élémentaire : | | | | | |
|---|---|---|---|---|---|
| | C % | H % | N % | Cl % | S % |
| calculé | 58,89 | 5,44 | 3,43 | 8,69 | 7,86 |
| trouvé | 58,63 | 5,39 | 3,58 | 8,87 | 7,53 |

### EXEMPLES 7a et 7b : isomères α et β du composé de l'exemple 7

Les stades A à H sont identiques aux stades A à H de l'exemple 7.

### Stade I : [6-Amino-2-méthyl-5,6,7,8-tétrahydronapht-1-yl]carboxylate d'éthyle

13 mmoles du composé obtenu au stade précédent en solution dans 50 ml de DMPU et 100 ml de tétrahydrofurane sont traitées par 80 mmoles d'iodure de samarium au reflux pendant 5 heures. Après évaporation des solvants, le produit est fixé sur une résine acide sulfonique, lavé à l'eau puis relargué à l'ammoniaque. Les deux énantiomères obtenus sont ensuite séparés classiquement à l'aide d'un acide chiral. Le sel d'acide est alors relargué. Chaque énantiomère subit alors la suite de réaction suivante :

### Stade J : [6-(4-Chlorophénylsulfonyl)amino-2-méthyl-5,6,7,8-tétrahydronapht-1-yl]carboxylate d'éthyle isomère α et isomère β

Le mode opératoire utilisé est identique à celui du stade A de l'exemple 5.

### Stades K, L, M et N : Ces stades sont identiques aux stades H, I, J et K de l'exemple 1.

### Stade O : Ce stade est identique au stade M de l'exemple 7.

### EXEMPLE 8 : Acide 3-[6-(1-napthylsulfonylamino)-2-méthyl-5,6,7,8-tétrahydronapht-1-yl]propionique

### Stade A : 4-(1-naphtylsulfonylamino)cyclohexanol

A 18,6 g de chlorhydrate de trans 4-aminocyclohexanol en suspension dans 700 ml de chloroforme sont additionnés, à 5°C, 34 ml de triéthylamine puis une solution contenant 18,6 g de chlorure de 1-naphtylsulfonyl dans 100 ml de chloroforme. Après 15 heures de réaction, le mélange réactionnel est versé sur 500 ml d'eau. Le produit attendu est obtenu par filtration du précipité.
- Rendement :: 89 %
- Point de fusion :: 174°C

### Stade B : 4-(1-Naphtylsulfonylamino)cyclohexanone

Le mode opératoire utilisé est identique au mode opératoire du stade B de l'exemple 5.
- Rendement :: 94 %

### Stades C, D et E : Les produits attendus dans ces stades sont obtenus selon les procédés décrits aux stades D, E et F de l'exemple 1.

### Stade F : [6-(1-Naphtylsulfonyl)amino-2-méthyl-5,6,7,8-tétrahydronapht-1-yl]carboxylate de méthyle

11,8 mmoles du composé obtenu au stade E dans 100 ml de décaline, on ajoute 48 mmoles de butynoate de méthyle. Le milieu réactionnel est chauffé en autoclave à 270°C pendant 6 h. Le produit est purifié par chromatographie sur colonne de silice en utilisant comme éluant un mélange cyclohexane/acétate d'éthyle (80/20).

### Stades G, H, I, J et K : Les produits attendus dans ces stades sont obtenus selon les procédés décrits aux stades I, J, K, L et M de l'exemple 7.

L'acide 3-[6-(1-naphtylsulfonylamino)-2-méthyl-5,6,7,8-tétrahydronapht-1-yl]propionique attendu est analysé par résonance magnétique nucléaire du proton en solution dans le CDCl₃. Sa pureté est vérifié par CLHP.

| Microanalyse élémentaire : | | | | |
|---|---|---|---|---|
| | C % | H % | N % | S % |
| calculé | 68,06 | 5,95 | 3,31 | 7,57 |
| trouvé | 67,50 | 6,13 | 3,39 | 7,83 |

### EXEMPLE 9 : Acide 3-[6-(4-tolylsulfonylamino)-5,6,7,8-tétrahydronapht-1-yl]propionique

Les stades A, B, C et D sont identiques aux stades A, B, C et D de l'exemple 6.

### Stade E : 3-[6-(4-Tolylsulfonylamino)-5,6,7,8-tétrahydronapht-1-yl]propanoate de méthyle

Le produit attendu est obtenu selon le mode opératoire décrit au stade E de l'exemple 6 en remplacant le chlorure de sulfonyle par le chlorure d'acide paratoluène sulfonique.

### Stade F : Acide 3-[6-(4-tolylsulfonylamino)-5,6,7,8-tétrahydronapht-1-yl]propionique

Le produit attendu est obtenu selon le procédé décrit au stade M de l'exemple 7.

| Microanalyse élémentaire : | | | | |
|---|---|---|---|---|
| | C % | H % | N % | S % |
| calculé | 64,32 | 6,21 | 3,75 | 8,59 |
| trouvé | 64,67 | 6,29 | 3,84 | 8,63 |

### EXEMPLE 10 : Acide 3-{6-[(4-chlorophénylsulfonyl)amino]3-méthyl-5,6,7,8-tétrahydronapht-1-yl}propionique

### Stade A : 2,3-Diméthyl-5,6,7,8-tétrahydrochrom-3-ène-5-one

Le produit attendu est obtenu sous forme d'huile selon le procédé décrit dans Tet. Lett., 39, 3407, 1975 par réaction de 0,99 mole de cyclohexan-1,3-dione et de 1,19 mole d'aldéhyde tiglique dans de la pyridine.

| Microanalyse élémentaire : | | |
|---|---|---|
| | C % | H % |
| calculé | 74,13 | 7,92 |
| trouvé | 73,97 | 7,71 |

### Stade B : (3-Méthyl-5-oxo-5,6,7,8-tétrahydronapth-1-yl)carboxylate d'éthyle

Le produit attendu est obtenu à partir du composé décrit au stade précédent et de propiolate d'éthyle selon le procédé décrit dans J. Org. Chem., 41, 2918, 1976.

| Microanalyse élémentaire : | | |
|---|---|---|
| | C % | H % |
| calculé | 72,39 | 6,94 |
| trouvé | 71,97 | 6,77 |

### Stade C : (3-Méthyl-5-hydroxyimino-5,6,7,8-tétrahydronapht-1-yl)carboxylate d'éthyle

Le produit attendu est obtenu selon le procédé décrit dans J. Med. Chem., 863, 1972 à partir du composé obtenu au stade précédent et d'hydroxylamine.

### Stade D : (3-Méthyl-5-tosyloxyimino-5,6,7,8-tétrahydronapth-1-yl)carboxylate d'éthyle

Le produit attendu est obtenu selon le procédé décrit dans J. Med. Chem., 863, 1972 à partir du composé décrit au stade précédent et de chlorure de tosyle.

### Stade E : (3-Méthyl-5-oxo-6-amino-5,6,7,8-tétrahydronapht-1-yl)carboxylate d'éthyle, chlorhydrate

A 142 mmoles de sodium dissoutes dans 66 ml d'éthanol et 730 ml de benzène, sont ajoutées 150 mmoles du composé obtenu au stade précédent. Après 20 heures d'agitation à température ambiante, le précipité est filtré et rincé à l'éther. Les phases organiques sont réunies et extraites par de l'acide chlorhydrique à 10 %. Après évaporation des phases aqueuses, on obtient le produit attendu sous forme d'un solide qui est rincé avec un mélange éthanol/éther (50/50).

### Stade F : [6-(4-Chlorophénylsulfonyl)amino-3-méthyl-5,6,7,8-tétrahydronapht-1-yl]carboxylate d'éthyle

35 mmoles du composé obtenu au stade précédent sont hydrogénées dans 80 ml d'acide acétique en présence de 3 g d'hydroxyde de palladium sur charbon à 10 %. Après absorption d'un équivalent d'hydrogène, 3 ml d'acide perchlorique à 70 % sont ajoutés et l'hydrogénolyse est poursuivie. Le catalyseur est alors filtré et 2,1 g d'acétate de potassium dans 20 ml d'acide acétique sont ajoutés au filtrat. Après filtration du précipité, le filtrat est évaporé. Le résidu obtenu est alors amidifié par addition de 35 mmoles de chlorure d'acide 4-chlorophénylsulfonique dans le chloroforme en présence de 70 mmoles de triéthylamine. Après évaporation, l'huile obtenue est purifiée par chromatographie sur colonne de silice en utilisant comme éluant un mélange dichlorométhane/méthanol (99/1).

### Stade G : 6-(4-Chlorophénylsulfonyl)amino-3-méthyl-1-hydroxyméthyl-5,6,7,8-tétrahydronaphtalène

Le produit attendu est obtenu selon le procédé décrit au stade H de l'exemple 1.

### Stade H : 6-(4-Chlorophénylsulfonyl)amino-3-méthyl-5,6,7,8-tétrahydronapht-1-yl-carboxaldéhyde

Le produit attendu est obtenu selon le procédé décrit au stade I de l'exemple 1.

### Stade I : 3-[6-(4-Chlorophénylsulfonyl)amino-3-méthyl-5,6,7,8-tétrahydronapht-1-yl]prop-2-ènoate de méthyle

Le produit attendu est obtenu selon le procédé décrit au stade J de l'exemple 1.

### Stade J : 3-[6-(4-Chlorophénylsulfonyl)amino-3-méthyl-5,6,7,8-tétrahydronapht-1-yl]propanoate de méthyle

Le produit attendu est obtenu selon le procédé décrit au stade K de l'exemple 1.

### Stade K : Acide 3-{6-[(4-chlorophénylsulfonyl)amino]3-méthyl-5,6,7,8-tétrahydronapht-1-yl}propionique

Le produit attendu est obtenu selon le procédé décrit au stade M de l'exemple 7.
- Point de fusion :: 170-173°C

| Microanalyse élémentaire : | | | | | |
|---|---|---|---|---|---|
| | C % | H % | N % | Cl % | S % |
| calculé | 58,89 | 5,44 | 3,43 | 8,69 | 7,88 |
| trouvé | 58,84 | 5,50 | 3,50 | 8,69 | 7,80 |

### EXEMPLE 11 : Acide 3-{6-[(4-fluorophénylsulfonyl)amino]-3-méthyl-5,6,7,8-tétrahydronapht-1-yl}propionique

Le produit attendu est obtenu selon le procédé décrit dans l'exemple 10 en remplaçant au stade F le chlorure d'acide 4-chlorophénylsulfonique par le chlorure d'acide 4-fluorophénylsulfonique.
- Point de fusion :: 174-176°C

| Microanalyse élémentaire : | | | | |
|---|---|---|---|---|
| | C % | H % | N % | S % |
| calculé | 61,37 | 5,66 | 3,58 | 8,19 |
| trouvé | 61,57 | 5,71 | 3,64 | 7,92 |

### EXEMPLE 12 : Acide 3-{6-[(4-chlorophénylsulfonyl)amino]-2-phényl-5,6,7,8-tétrahydronapht-1-yl]propionique

### Stades A à F : Ces stades sont identiques aux stades A à F de l'exemple 1.

### Stade G : [6-(4-Chlorophénylsulfonyl)amino-2-tributylstannanyl-5,6,7,8-tétrahydronapht-1-yl]carboxylate de méthyle

Le produit attendu est obtenu selon le procédé décrit au stade G de l'exemple 1 en remplaçant le but-2-ynoate de méthyle par le 3-tributylstannanyl propynoate de méthyle.

### Stade H : [6-(4-Chlorophénylsulfonyl)amino-2-phényl-5,6,7,8-tétrahydronapth-1-yl] carboxylate de méthyle

A 1 g du composé obtenu au stade précédent dissous dans 20 ml de N-méthylpyrrolidone, on ajoute successivement 750 µl de bromobenzène et 80 mg de tétrakistriphénylphosphine palladium. Le milieu réactionnel est porté à 110°C pendant 16 heures. Après évaporation du solvant et chromatographie sur colonne de silice en utilisant comme éluant un mélange cyclohexane/acétate d'éthyle (80/20), on obtient le produit attendu.

### Stades I à M : Ces stades sont identiques aux stades I à M de l'exemple 7 et conduisent au produit du titre.

- Point de fusion :: 98-100°C

| Microanalyse élémentaire : | | | | | |
|---|---|---|---|---|---|
| | C % | H % | N % | Cl % | S % |
| calculé | 63,89 | 5,15 | 2,98 | 7,54 | 6,82 |
| trouvé | 63,61 | 5,15 | 3,03 | 8,45 | 6,43 |

### EXEMPLE 13 : Acide 3-{6-[(4-chlorophénylsulfonyl)amino]-2-isopropyl-5,6,7,8-tétrahydronapht-1-yl}propionique

Le produit attendu est obtenu selon le procédé décrit dans l'exemple 1 en remplaçant le but-2-ynoate de méthyle par le 4-méthylpent-2-ynoate de méthyle. L'acide libre est directement obtenu au stade L par extraction avec de l'acétate d'éthyle.
- Point de fusion :: 167-169°C

| Microanalyse élémentaire : | | | | | |
|---|---|---|---|---|---|
| | C % | H % | N % | Cl % | S % |
| calculé | 60,61 | 6,01 | 3,21 | 8,13 | 7,35 |
| trouvé | 60,60 | 5,95 | 3,30 | 8,20 | 6,76 |

### EXEMPLE 14 : Acide 3-{6-[(4-chlorophénylsulfonyl)amino]-3-(pyridin-3-yl)méthyl-5,6,7,8-tétrahydronapht-1-yl}propionique

Les stades A à F sont identiques aux stades A à F de l'exemple 1.

### Stade G : 3-Bromo-6-(4-chlorophénylsulfonyl)amino-2-oxo-5,6,7,8-tétrahydro-2H-benzo[e]pyrane

Le produit attendu est obtenu selon le procédé décrit dans Synthesis, 34, 8, 2239-2244, 1969. 50 g du composé obtenu au stade F sont mis en suspension dans 500 ml d'acide acétique. Le mélange est agité vigoureusement et 8 ml de brome sont ajoutés. Après 4 heures d'agitation à température ambiante, le solvant est évaporé. Le résidu est purifié par une chromatographie sur colonne de silice avec comme éluant un mélange cyclohexane/acétate d'éthyle (50/50).
- Point de fusion :: 168-170°C

### Stade H : [3-Bromo-6-(4-chlorophénylsulfonyl)amino-5,6,7,8-tétrahydronapht-1-yl]carboxylate de méthyle

5 g du composé obtenu au stade précédent sont placés dans un autoclave avec 5 ml de propynoate de méthyle et 80 ml de décaline et l'ensemble est porté 16 heures à 200°C. Le produit attendu est alors obtenu après purification par chromatographie sur colonne de silice en utilisant comme éluant un mélange cyclohexane/acétate d'éthyle (80/20).

### Stade I : 3-Bromo-6-(4-chlorophénylsulfonyl)amino-1-hydroxyméthyl-5,6,7,8-tétrahydronaphtalène

Le produit attendu est obtenu selon le procédé décrit au stade H de l'exemple 1.

### Stade J : 3-Bromo-6-(4-chlorophénylsulfonyl)amino-5,6,7,8-tétrahydronapht-1-yl carboxaldéhyde

Le mode opératoire est identique à celui décrit au stade I de l'exemple 1.

### Stade K : 6-(4-Chlorophénylsulfonyl)amino-3-(pyridin-3-yl)méthyl-5,6,7,8-tétrahydronapht-1-yl-carboxaldéhyde

A 1,57 g du composé obtenu au stade précédent dissous dans 10 ml de N-méthylpyrrolidone, on ajoute successivement 3,75 g de 3-tributylstannanylméthyl-pyridine, et 0,4 g de tétrakis triphénylphosphine palladium. Le milieu réactionnel est porté à 110°C pendant 7 heures. Après évaporation du solvant et chromatographie sur colonne de silice avec comme éluant un mélange cyclohexane/acétate d'éthyle (40/60), on obtient le produit attendu.

### Stade L : 3-[6-(4-Chlorophénylsulfonyl)amino-3-(pyridin-3-yl)méthyl-5,6,7,8-tétrahydronapht-1-yl]prop-2-ènoate de méthyle

A 930 mg du composé obtenu au stade précédent dissous dans 25 ml de dichlorométhane, on ajoute 800 mg de (carbométhoxyméthylidène)triphénylphosphorane. Après 48 heures, à température ambiante, le solvant est évaporé et le produit attendu est obtenu après purification par chromatographie sur colonne de silice en utilisant comme éluant un mélange cyclohexane/acétate d'éthyle (50/50).

### Stade M : 3-[6-(4-Chlorophénylsulfonyl)amino-3-(pyridin-3-yl)méthyl-5,6,7,8-tétrahydronapht-1-yl]propanoate de méthyle

A 800 mg du composé obtenu au stade précédent dissous dans 20 ml de méthanol, on ajoute à température ambiante successivement 95 mg de chlorure de cobalt hexahydrate puis par petites portions 121 mg de borohydrure de sodium. Après agitation de 2 heures, le solvant est évaporé. Le produit attendu est purifié sur colonne de silice en utilisant comme éluant un mélange cyclohexane/acétate d'éthyle (50/50).

### Stade N : Acide 3-{6-[(4-chlorophénylsulfonyl)amino]-3-(pyridin-3-yl)méthyl-5,6,7,8-tétrahydronapht-1-yl}propionique

A 610 mg du produit obtenu au stade précédent dissous dans 50 ml de méthanol, on ajoute 4 ml de soude 1N. Le mélange est porté au reflux pendant 2 heures. Après refroidissement de la solution, de l'acide acétique est ajouté jusqu'à pH = 6. Le produit attendu est alors obtenu par filtration.

### EXEMPLE 15 : Acide 3-{6-[(4-chlorophénylsulfonyl)amino]-2-méthyl-3-(pyridin-3-yl)méthyl-5,6,7,8-tétrahydronapht-1-yl}propionique

Le produit attendu est obtenu selon le procédé décrit dans l'exemple 14 en remplaçant au stade H le propynoate de méthyle par le but-2-ynoate de méthyle.

### EXEMPLE 16 : Acide 3-[3-(4-chlorophénylsulfonyl)amino-chroman-8-yl]propionique

### Stade A : Acide 3-(2-bromophénoxy)propionique

A 1 mole de sel de potassium de l'acide 3-bromopropionique et 1 mole de 2-bromophénate de potassium sont portées à reflux dans 1 litre d'éthanol et 200 ml d'eau. Après évaporation des solvants et reprise de résidu par de l'eau, le pH est amené à 7,2. La phase aqueuse est lavée par de l'acétate d'éthyle, acidifiée et le produit attendu est obtenu par filtration du précipité formé.

### Stade B : 3-Bromochroman-4-one

326 mmoles du composé obtenu au stade précédent sont chauffées à 100°C en présence de 500 g d'acide polyphosphonique. 1,5 kg de glace sont alors ajoutés au mélange et après extraction à l'acétate d'éthyle et évaporation, on obtient le produit attendu.
- Point de fusion :: 48-51°C

### Stade C : 3-(4-Oxochroman-8-yl)prop-2-ènoate de méthyle

20 mmoles du composé obtenu au stade précédent, 24,5 ml d'acrylate de méthyle, 300 ml de triéthylamine, 0,6 g d'acétate de palladium et 10 mmoles d'triorthotolylphosphine sont portés 10 h à 100°C. Après concentration sous vide, le résidu est repris par du dichlorométhane. La phase organique est lavée par de l'acide chlorhydrique 1N puis par de l'eau, évaporée et conduit au produit attendu.

### Stades D, E et F : Ces stades sont identiques aux stades C, D et E de l'exemple 10 et conduisent au 3-(3-amino-4-oxo-chroman-8-yl)prop-2-ènoate de méthyle.

### Stade G : 3-[3-(4-Chlorophénylsulfonyl)amino-chroman-8-yl]propanoate de méthyle

Le composé obtenu au stade précédent est hydrogéné dans de l'acide acétique à 70°C à une pression de 3 bars en présence de palladium sur charbon. Lorsque 2 équivalents d'hydrogène ont été absorbés, on a,joute de l'acide perchlorique à 70°C et l'hydrogénolyse est poursuivie. Le produit est alors traité dans les mêmes conditions que celles décrites au stade F de l'exemple 10.

### Stade H : Acide 3-[3-(4-chlorophénylsulfonyl)amino-chroman-8-yl]propionique

Le produit attendu est obtenu par saponification du composé décrit au stade précédent dans les conditions décrites au stade K de l'exemple 10.

| Microanalyse élémentaire : | | | | |
|---|---|---|---|---|
| | C % | H % | N % | S % |
| calculé | 54,61 | 4,58 | 3,54 | 8,10 |
| trouvé | 54,51 | 4,50 | 3,68 | 7,90 |

### EXEMPLE 17 : Acide -3-[3-(4-fluorophénylsulfonyl)amino-chroman-8-yl]propionique

Le produit attendu est obtenu selon le procédé décrit dans l'exemple 16 en utilisant le chlorure d'acide 4-fluorophénylsulfonique.

| Microanalyse élémentaire : | | | | |
|---|---|---|---|---|
| | C % | H % | N % | S % |
| calculé | 56,98 | 4,78 | 3,69 | 8,45 |
| trouvé | 56,46 | 5,03 | 3,60 | 8,55 |

### Etude pharmacologique des dérivés de l'invention

### EXEMPLE 18 : Agrégation plaquettaire chez le lapin

Les lapins (2-3 kg) sont anesthésiés avec du sodium pentobarbital (30 mg/kg i.v.). Après cannulation de l'artère carotide gauche, le sang est prélevé sur citrate de sodium (0.109 M) (1 vol. de citrate pour 9 vol. de sang).
Le plasma riche en plaquettes (PRP) est obtenu par centrifugation (20°C) à 250 g pendant 20 minutes et le plasma pauvre en plaquettes (PPP) par centrifugation à 1000 g (10 min). Le nombre des plaquettes (PL) dans le PRP est ajusté entre 300-350000 PL/mm³ par dilution avec du PPP autologue. Le PRP est conservé à la température de la pièce jusqu'au moment du test et est utilisé dans les 4 heures qui suivent le prélèvement.
L'agrégation plaquettaire est réalisée à 37°C dans des tubes en verre siliconés à l'aide d'un agrégomètre. Le PRP et les PL sont agités à 1000 rpm (révolution par minute). Afin d'étudier l'activité des antagonistes du thromboxane, le PRP est incubé 1 min à 37°C, puis l'antagoniste est ajouté pour une durée de 3 min avant l'addition de l'agoniste U46619 (1 µM). Le volume final de la cuve est alors de 250 µl. L'intensité de l'agrégation plaquettaire est établie en prenant l'amplitude maximale de tracés agrégants et est exprimée en pourcentage de transmission lumineuse (% T). L'activité des antagonistes est exprimée en IC₅₀, c'est-à-dire la concentration de la substance qui induit 50 % d'inhibition de la réponse agrégante induite par le U46619.
Les composés de l'invention inhibent l'agrégation plaquettaire induite par l'agoniste TXA₂, le U46619. Les IC₅₀ illustrées dans le tableau ci-dessous montrent que les composés de l'invention ont une activité égale ou supérieure à celle du composé de référence le BAY U3405.

| **Exemple** | **IC**_{**50**} **(µM) Lapin** |
|---|---|
| ex. 1 | 0.24 |
| ex. 2 | 0.82 |
| ex. 3 | 0.27 |
| ex. 4 | 0.16 |
| ex. 6 | 0.060 |
| ex. 9 | 0.32 |
| ex. 10 | 0.18 |
| ex. 11 | 0.67 |
| ex. 12 | 0.097 |
| ex. 13 | 0.16 |
| ex. 16 | 0.25 |
| ex. 17 | 1.1 |
| BAY U3405 | 1.10 |

### EXEMPLE 19 : Agrégation plaquettaire chez le chien :

Après anesthésie de l'animal par le sodium pentobarbital (30 mg/kg i.v.), le sang artériel est prélevé sur citrate de sodium (0,109 M) (1 vol. de citrate pour 9 vol. de sang). Le plasma riche en plaquettes (PRP) est obtenu après centrifugation (20°C) à 200 g pendant 10 minutes. Le nombre de plaquettes dans le PRP est en moyenne de 300 000 PL/mm³. Le PRP est conservé à la température de la pièce jusqu'au moment du test et est utilisé dans les 4 heures qui suivent le prélèvement.
Les plaquettes du chien répondent faiblement au U46619 seul. L'addition d'adrénaline, qui seule n'induit pas d'agrégation, permet d'obtenir une réponse agrégante plus importante au U46619. Le PRP est incubé à 37°C en présence de l'antagoniste à tester pendant 3 minutes. L'agrégation est ensuite obtenue par l'addition de l'adrénaline (10 µM) suivie de celle du U46619 (1 µM) 30 secondes après. L'effet des antagonistes est mesuré et l'IC₅₀ est déterminée comme la concentration de l'antagoniste nécessaire pour produire 50 % d'inhibition des réponses agrégantes au U46619 + adrénaline.

Les composés de l'invention inhibent l'agrégation plaquettaire induite par l'agoniste TXA₂,le U46619. Les IC₅₀ illustrées dans le tableau ci-dessous montrent que les composés de l'invention ont une activité égale ou supérieure à celle du composé de référence le BAY U3405.

| **Exemple** | **IC**_{**50**} **(µM) Chien** |
|---|---|
| ex. 1 | 0.028 |
| ex. 2 | 0.083 |
| ex. 3 | 0.010 |
| ex. 4 | 0.010 |
| ex. 6 | 0.003 |
| ex. 8 | 0.15 |
| ex. 9 | 0.015 |
| ex. 10 | 0.011 |
| ex. 11 | 0.056 |
| ex. 12 | 0.012 |
| ex. 13 | 0.042 |
| ex. 16 | 0.020 |
| BAY U3405 | 0.110 |

### EXEMPLE 20 : Agrégation plaquettaire chez l'homme :

Le sang veineux est obtenu de volontaires humains n'ayant pas pris de l'aspirine pendant au moins 14 jours précédent l'expérience. Le sang est prélevé sur citrate de sodium (0,109 M) (1 vol. de citrate sur 9 vol. de sang). Le plasma riche en plaquettes (PRP) est obtenu après centrifugation (20°C) à 200 g pendant 10 minutes. Le nombre de plaquettes est en moyenne de 250000 PL/mm³. Le PRP est conservé à la température de la pièce jusqu'au moment du test et est utilisé dans les 2 heures qui suivent le prélèvement. Les antagonistes sont testés suivant la procédure décrite dans l'exemple 19 en utilisant le U46619 à la concentration de 0,3 µM.
Les composés de l'invention inhibent l'agrégation plaquettaire induite par l'agoniste TXA₂, le U46619. L'IC₅₀ du composé de l'exemple 1 est de 84 nM alors que celle du produit de référence le BAY U3405 est de 180 nM.

### EXEMPLE 21 : Liaison spécifique sur des membranes de plaquettes humaines

Les essais de liaison spécifique sur les récepteurs TXA₂ plaquettaires se font en utilisant comme ligand le ³H-SQ29548 (selon le protocole décrit par A. Hedberg et coll. J. Pharm. Exp. Ther., 245, 786-792, 1988). Les expériences ont lieu à une température de 25°C dans un volume réactionnel final de 0,2 ml en présence de 0,1 mg de membranes plaquettaires préparées à partir de plaquettes humaines lavées, broyées et centrifugées. La détermination de l'activité des produits de l'invention se fait par des expériences de compétition, dans lesquelles des concentrations croissantes de produit sont mises en présence d'une concentration fixe du ³H-SQ29548. Ce protocole permet de réaliser des courbes d'inhibition pour chaque antagoniste étudié. La concentration inhibitrice 50 % est déterminée par régression non-linéaire par la méthode "Simplex" décrite par M.S. CACECI and coll. (BYTE, 340-362, Mai 1984) calculée suivant le modèle de la loi d'action de masse de C. Michaelis et coll. (Biochem. Zeitschrifft, 49, 333-369, 1913). La constante d'inhibition est alors déterminée en utilisant la formule de Cheng et Prusoff (Biochem. Pharmacol., 22, 3099-3108, 1973). Les substances de l'invention inhibent la liaison spécifique du ³H-SQ29548. Le Ki du composé de l'exemple 1 est de 0,96 nM démontrant une forte affinité pour les récepteurs TXA₂ plaquettaires. Cette activité est plus importante que celle de la référence, le BAY U3405, qui présente un Ki de 3,61 nM dans ce même test.

### EXEMPLE 22 : Agrégation plaquettaire ex vivo chez le chien

Les expériences sont effectuées chez le chien non-anesthésié. Après la pose d'un garrot et introduction d'une aiguille dans la veine céphalique, le sang est prélevé sur citrate de sodium (0,109 M). Le sang est conservé à la température de la pièce. L'agrégation plaquettaire est mesurée dans le sang total à l'aide d'une sonde à impédance dans un agrégomètre. L'activité proagrégante de l'agoniste des récepteurs au thromboxane, le U46619 est testée en présence d'adrénaline (10 µM). Les produits de l'invention ainsi que les substances de référence sont administrés aux chiens par voie orale après la réalisation du test contrôle. Des prélèvements sanguins sont ensuite réalisés à des temps déterminés : t = 30 min, 1 h, 2 h, 4 h, 6 h, 24 h, 48 h... jusqu'au retour complet de l'activité agrégante du U46619. Les produits de l'invention inhibent de façon complète, l'agrégation plaquettaire ex vivo induite par le U46619. Le composé de l'exemple 1 à des doses de 10 à 3000 µg/kg inhibe complètement l'agrégation plaquettaire au U46619 pendant au moins 3 jours, et pour la plus forte dose, jusqu'à 11 jours. Après, l'activité proagrégante du U46619 revient progressivement. Le composé de l'exemple 1 à la dose de 10 µg/kg per os, inhibe complètement l'agrégation plaquettaire au U 46619 pendant au moins 3 jours. A la plus forte dose de 100 µg/kg per os, il inhibe l'agrégation plaquettaire au U 46619 pendant au moins 8 jours. Un même résultat a été obtenu avec 3 mg/kg per os de ce composé. Le composé de l'exemple 10 à la dose de 100 µg/kg per os inhibe l'agrégation plaquettaire induite par le U 46619 pendant 5 jours. L'effet anti-agrégant des composés de l'invention est plus durable que celui observé avec le BAY U3405. En effet, à la dose de 100 µg/kg per os, l'inhibition complète de l'agrégation obtenue avec le BAY U3405 ne dure que 6 heures. Les expériences montrent que les composés de l'invention sont bien absorbées per or et ont une durée d'action très importante au niveau des récepteurs plaquettaires au TXA₂.

### Exemple 23 : Thrombose expérimentale dans l'artère carotide de cobaye

La technique décrite récemment par Roux et al., Thrombosis and Haemostasis, 71 : 252-256 (1994) a été utilisée afin de mesurer l'effet antithrombotique de nos produits. Des cobayes mâles (390 à 420 g) ont été anesthésié par la kétamine hydrochloride (90 mg/kg i.m.) et la xylazine (12 mg/kg i.m.). Un cathéter est introduit dans la veine jugulaire gauche afin de permettre l'injection i.v. des substances. L'artère carotide droite est préparée et une probe Doppler (20 MHz) est installée permettant la mesure du flux sanguin. A deux mm distale de la probe Doppler, une lésion subendothéliale est produite à l'aide d'une pince ("pinching"). Après l'introduction de cette lésion, le flux diminue et s'arrête complètement (entre 1 et 2 min). quand le flux est à zéro, on touche l'artère ce qui élimine le thrombus occlusif et le flux est restauré (voir Roux et al., 1994). Ce processus thrombotique se répète et on exprime les diminutions cycliques du flux (CFV = cyclic flow variations) en nombre par 20 min. Dans des artères carotides témoins (n=4), les CFV sont compté durant 2 périodes de 20 min : pendant la première période, on compte 9 ± 1 CFV / 20 min, pendant le deuxième période, on compte 8 ± 1 CFV / 20 min. Dans un groupe d'animaux (n=3) traité avec le composé de l'exemple 1, on compte 10 ± 2 CFV / 20 min avant le traitement et 0.3 ± 0.3 CFV / 20 min après une injection i.v. de 100 µg/kg. Ces résultats démontrent un effet antithrombotique puissant.

### EXEMPLE 24 : Composition pharmaceutique

Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 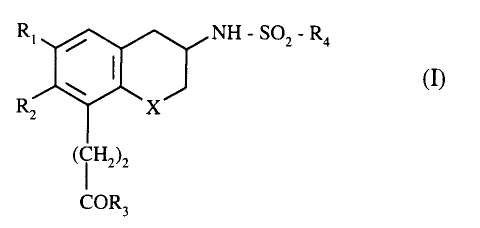 dans laquelle :
R₁, R₂, identiques ou différents, représentent un atome d'hydrogène, d'halogène, un groupement alkyle (C₁-C₆) linéaire ou ramifié, un groupement phényle (substitué ou non par un ou plusieurs atomes d'halogène ou groupements alkyl (C₁-C₆) linéaire ou ramifié, alkoxy (C₁-C₆) linéaire ou ramifié ou trihalogénométhyle), un groupement benzyle, un groupement pyridylméthyle ou un groupement imidazolylméthyle, thiazolylméthyle, pyridyle, imidazolyl, thiazolyl,
ou bien,
R₁ et R₂ forment avec les atomes de carbone auxquels ils sont attachés un cycle cyclopentane ou cyclohexane,
R₃ représente un groupement hydroxy, un groupement alkoxy (C₁-C₆) linéaire ou ramifié ou amino (substitué ou non par un ou deux groupements alkyle (C₁-C₆) linéaire ou ramifié),
R₄ représente un groupement alkyle (C₁-C₆) linéaire ou ramifié, un groupement phényle (substitué ou non par un ou plusieurs atomes d'halogène ou groupements alkyle (C₁-C₆) linéaire ou ramifié, alkoxy (C₁-C₆) linéaire ou ramifié, trihalogénométhyle ou hydroxy), un groupement naphtyle, un groupement pyridyle, un groupement thiényle ou un groupement thiazolyle,
X représente un groupement méthylène, un atome d'oxygène ou de soufre,
leurs énantiomères, ainsi que leurs sels d'addition à une base pharmaceutiquement acceptable.

2. Composés de formule (I) selon la revendication 1 dans laquelle R₁ représente un atome d'hydrogène ou un groupement méthyle.

3. Composés de formule (I) selon la revendication 1 dans laquelle R₂ représente un groupement méthyle.

4. Composés de formule (I) selon la revendication 1 dans laquelle X représente un groupement méthylène.

5. Composés de formule (I) selon la revendication 1 qui est l'acide {6-[(4-chlorophénylsulfonyl)amino]-2-méthyl-5,6,7,8-tétrahydronapht-1-yl} propionique, ainsi que ses sels d'addition à une base pharmaceutiquement acceptable.

6. Procédé de préparation des composés de formule (I) selon la revendication 1 dans laquelle X représente un groupement méthylène caractérisé en ce que l'on utilise comme produit de départ un chlorure de sulfonyle de formule (II) :
R₄ - SO₂Cl (II)
dans laquelle R₄ a la même signification que dans la formule (I),
que l'on fait réagir en milieu basique :
soit sur une cyclohexylamine de formule (III) (préparée à partir de la cyclohexanone correspondante par réaction, sous atmosphère inerte, avec la benzylamine en présence de triacétoxyborohydrure de sodium puis hydrogénolyse) : 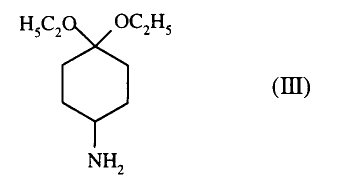 pour conduire au composé de formule (IV) : 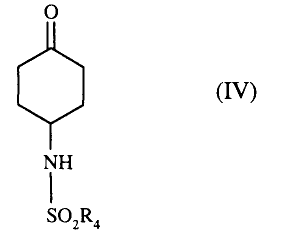 dans laquelle R₄ a la même signification que dans la formule (I),
soit sur le 4-aminocyclohexanol,
pour conduire au composé de formule (V) : 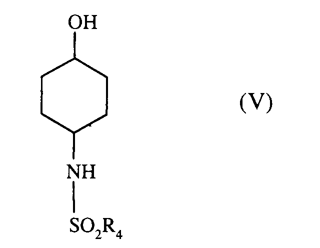 dans laquelle R₄ a la même signification que dans la formule (I),
que l'on oxyde à l'aide du réactif de Jones (acide chromique dans l'acétone et l'acide sulfurique aqueux),
pour conduire au composé de formule (IV) précédemment décrit, composé de formule (IV),
que l'on fait réagir avec du formiate d'éthyle en présence d'hydrure de sodium,
pour conduire au composé de formule (VI) : 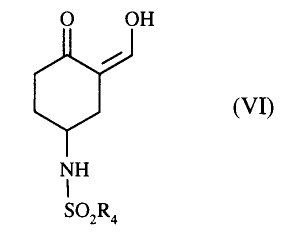 dans laquelle R₄ a la même signification que dans la formule (I),
qui subit ensuite, selon la nature des composés de formule (I) que l'on souhaite obtenir, l'action d'un carbalkoxyméthylènetriphénylphosphorane éventuellement substitué en α du groupe ester par un atome d'halogène, un groupement alkyle (C₁-C₆) linéaire ou ramifié, un groupement phényle (substitué ou non par un ou plusieurs atomes d'halogène ou groupement alkyl(C₁-C₆) linéaire ou ramifié, alkoxy (C₁-C₆) linéaire ou ramifié ou trihalogénométhyle), un groupement benzyle, un groupement pyridylméthyle ou un groupement imidazolylméthyle, pour conduire au composé de formule (VII) : 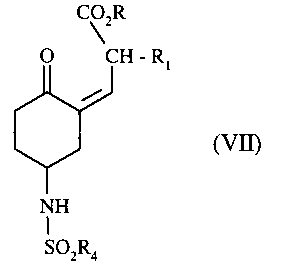 dans laquelle R₁ et R₄ ont la même signification que dans la formule (I) et R représente un groupement alkyle (C₁-C₆) linéaire ou ramifié,
que l'on met ensuite en réaction avec de l'acide p.toluènesulfonique ou l'acide trifluoroacétique,
pour conduire au composé de formule (VIII) : 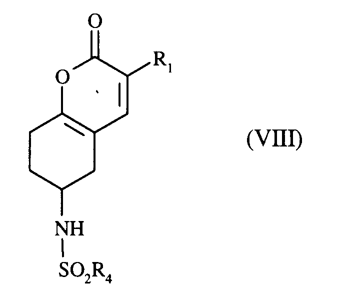 dans laquelle R₁ et R₄ ont la même signification que dans la formule (I),
qui subit une réaction de Diels-Alder avec un alc-2-ynoate d'alkyle convenablement substitué,
pour conduire après transformation éventuelles au composé de formule (IX) : 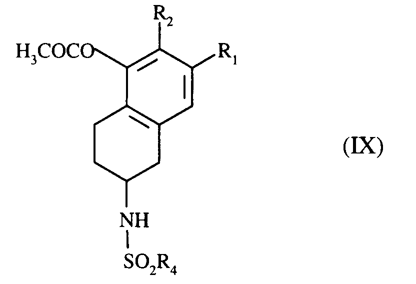 dans laquelle R₁, R₂ et R₄ ont la même signification que dans la formule (I),
qui subit l'action de l'hydrure de lithium aluminium en milieu anhydre,
pour conduire au composé de formule (X) : 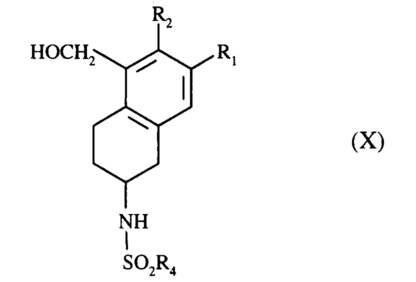 dans laquelle R₁, R₂ et R₄ ont la même signification que dans la formule (I),
que l'on fait réagir avec un agent d'oxydation comme le 4-benzylpyridinium dichromate,
pour conduire à l'aldéhyde de formule (XI) : 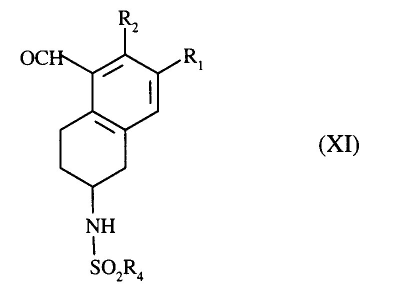 dans laquelle R₁, R₂ et R₄ ont la même signification que dans la formule (I),
que l'on fait réagir avec du (carbométhoxyméthylène)triphénylphosphorane,
pour conduire au composé de formule (XII) : 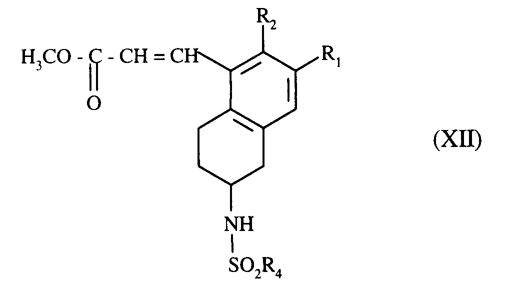 dans laquelle R₁, R₂ et R₄ ont la même signification que dans la formule (I),
que l'on réduit à l'aide d'iodure de samarium en présence de méthanol,
pour conduire au composé de formule (XIII) : 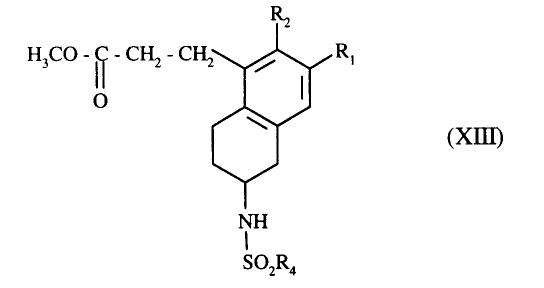 dans laquelle R₁, R₂ et R₄ ont la même signification que dans la formule (I),
que l'on transforme, ensuite en acide, ester ou amide correspondant, selon une technique classique de la chimie organique,
pour conduire au composé de formule (I/a), cas particulier des composés de formule (I) : 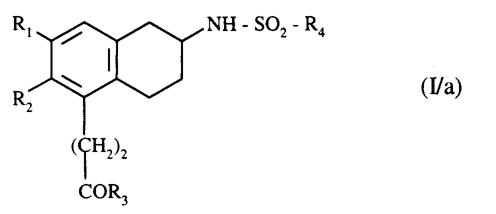 dans laquelle R₁, R₂, R₃ et R₄ ont la même signification que dans la formule (I),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

7. Procédé de préparation des composés de formule (I) selon la revendication 1 dans laquelle R₁ et R₂ représentent un atome d'hydrogène et X un groupement méthylène caractérisé en ce que l'on utilise comme produit de départ un composé de formule (XIV) : 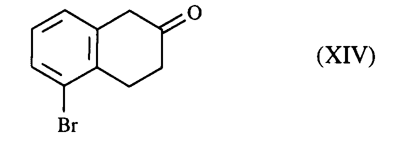 que l'on fait réagir avec un excès d'acrylate de méthyle en présence d'une quantité catalytique d'acétate de palladium et de triorthotolylphosphine dans la triéthylamine,
pour conduire au composé de formule (XV) : 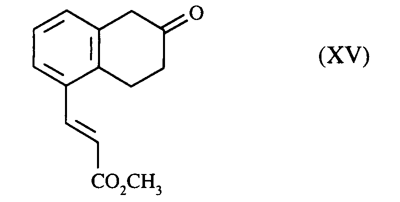 que l'on transforme en composé de formule (XVI) par réaction avec la benzylamine en présence de triacétoxyborohydrure de sodium, 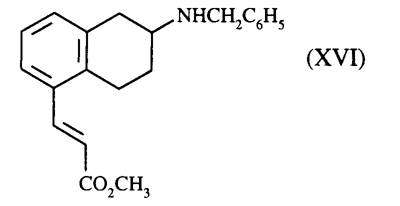 qui subit une hydrogénation catalytique pour conduire au composé de formule (XVII) : 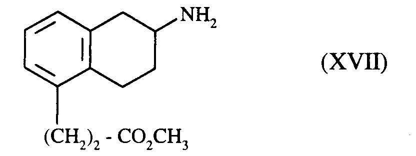 sur lequel on fait réagir un chlorure de sulfonyle de formule (II), en milieu basique :
R₄SO₂Cl (II)
dans laquelle R₄ a la même signification que dans la formule (I),
pour conduire au composé de formule (XVIII) : 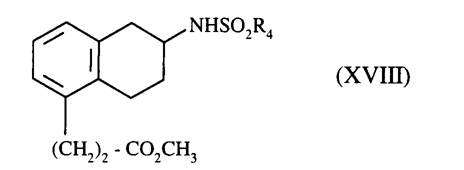 dans laquelle R₄ a la même signification que dans la formule (I),
que l'on transforme, ensuite en acide, ester ou amide correspondant, selon une technique classique de la chimie organique,
pour conduire au composé de formule (I/b), cas particulier des composés de formule (I) : 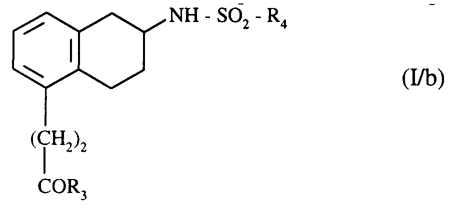 dans laquelle R₃ et R₄ ont la même signification que dans la formule (I),
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

8. Procédé de préparation des composés de formule (I) selon la revendication 1 dans laquelle X = X' représente un atome de soufre ou d'oxygène, caractérisé en ce que l'on utilise comme produit de départ un composé de formule (XIX) : 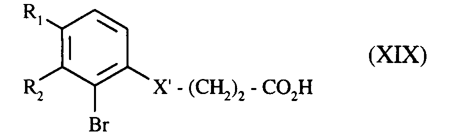 dans laquelle R₁ et R₂ ont la même signification que dans la formule (I) et X' représente un atome de soufre ou d'oxygène,
que l'on cyclise en présence d'acide polyphosphorique pour conduire au composé de formule (XX) : 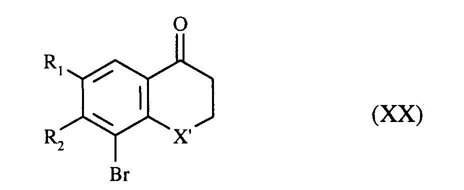 dans laquelle R₁, R₂ et X' ont la même signification que précédemment,
que l'on fait réagir avec un excès d'acrylate de méthyle en présence d'une quantité catalytique d'acétate de palladium et de triorthotolylphosphine dans la triéthylamine,
pour conduire au composé de formule (XXI) : 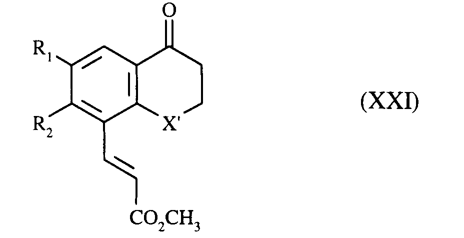 dans laquelle R₁, R₂ et X' ont la même signification que précédemment,
que l'on fait réagir avec de l'hydroxylamine, puis avec du chlorure de tosyle et enfin qui subit la transformation de Néber pour conduire au composé de formule (XXII) : 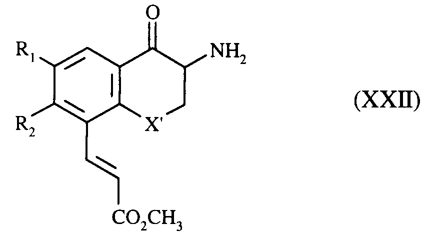 dans laquelle R₁, R₂ et X' ont la même signification que précédemment,
qui subit alors une réduction en présence d'un catalyseur, pour conduire au composé de formule (XXIII), 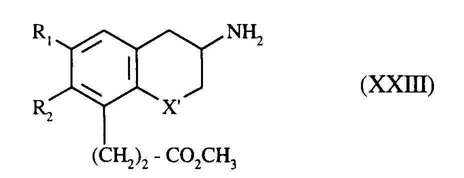 dans laquelle R₁, R₂ et X' ont la même signification que précédemment,
sur lequel on fait réagir un chlorure de sulfonyle de formule (II), en milieu basique :
R₄SO₂Cl (II)
dans laquelle R₄ a la même signification que dans la formule (I),
pour conduire au composé de formule (XXIV) : 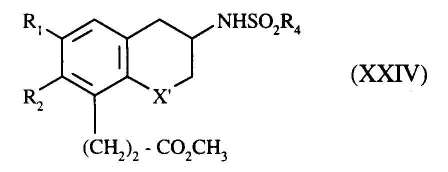 dans laquelle R₁, R₂, R₄ et X' ont la même signification que précédemment,
que l'on transforme, ensuite en acide, ester ou amide correspondant, selon une technique classique de la chimie organique,
pour conduire au composé de formule (I/c), cas particulier des composés de formule (I) : 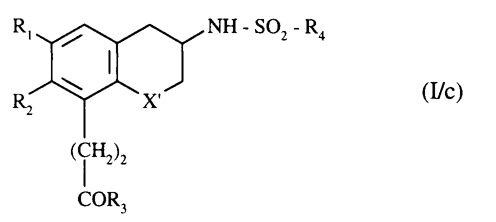 dans laquelle R₁, R₂, R₃, R₄ et X' ont la même signification que précédemment,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

9. Procédé de préparation des composés de formule (I) selon la revendication 1 dans laquelle X représente un groupement méthylène, R₁ représente un groupement méthyle et R₂ représente un atome d'hydrogène caractérisé en ce que l'on utilise comme produit de départ la 1,3-cyclohexanedione que l'on fait réagir avec l'aldéhyde tiglique pour conduire au composé de formule (XXV) : 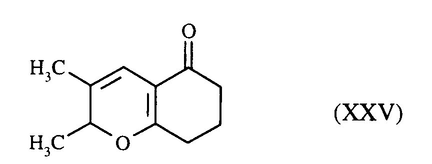 que l'on fait réagir avec du propiolate d'alkyle pour conduire au composé de formule (XXVI) : 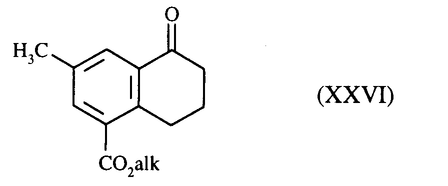 dans laquelle alk représente un groupement alkyle, que l'on fait réagir avec de l'hydroxylamine, puis avec du chlorure de tosyle et enfin qui subit une transformation de Neber,
pour conduire au composé de formule (XXVII) : 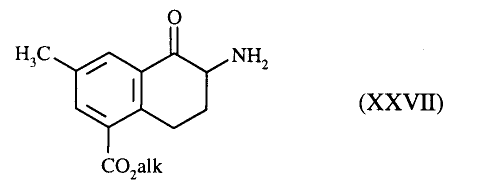 dans laquelle alk a la même signification que précédemment, qui subit alors une réduction catalytique, puis l'action du chlorure du sulfonyle de formule (II) selon la revendication 6,
pour conduire au composé de formule (XXVIII) : 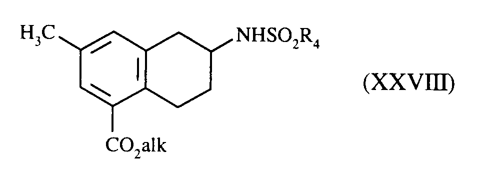 dans laquelle R₄ et alk ont la même signification que précédemment,
composé de formule (XXVIII) qui subit alors la suite de réactions selon la revendication 6 décrite pour la transformation du composé de formule (IX) en composé de formule (I/a) et qui conduit au composé de formule (I) correspondant.

10. Compositions pharmaceutiques contenant comme principe actif au moins un composé selon l'une quelconque des revendications 1 à 5, seul ou en combinaison avec un ou plusieurs véhicules inertes non toxiques pharmaceutiquement acceptables.

11. Composition pharmaceutique selon la revendication 10 contenant au moins un principe actif selon l'une des revendications 1 à 5 utile en tant qu'antithrombotique.

## Claims

1. Compounds of formula (I) : 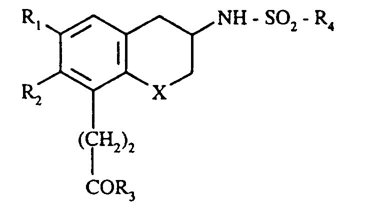 wherein :
R₁, R₂, which are identical or different, each represents a hydrogen atom, a halogen atom, a straight-chain or branched (C₁-C₆)-alkyl group, a phenyl group (unsubstituted or substituted by one or more halogen atoms or straight-chain or branched (C₁-C₆)-alkyl, straight-chain or branched (C₁-C₆)-alkoxy or trihalomethyl groups), a benzyl group, a pyridylmethyl group, or an imidazolylmethyl, thiazolylmethyl, pyridyl, imidazolyl or thiazolyl group,
or
R₁ and R₂ form together with the carbon atoms to which they are attached a cyclopentane or cyclohexane ring,
R₃ represents a hydroxy group, a straight-chain or branched (C₁-C₆)-alkoxy group or amino (unsubstituted or substituted by one or two straight-chain or branched (C₁-C₆)-alkyl groups),
R₄ represents a straight-chain or branched (C₁-C₆)-alkyl group, a phenyl group (unsubstituted or substituted by one or more halogen atoms or straight-chain or branched (C₁-C₆)-alkyl, straight-chain or branched (C₁-C₆)-alkoxy, trihalomethyl or hydroxy groups), a naphthyl group, a pyridyl group, a thienyl group or a thiazolyl group,
X represents a methylene group or an oxygen or sulphur atom,
the enantiomers thereof, and also the addition salts thereof with a pharmaceutically acceptable base.

2. Compounds of formula (I) according to claim 1, wherein R₁ represents a hydrogen atom or a methyl group.

3. Compounds of formula (I) according to claim 1 wherein R₂ represents a methyl group.

4. Compounds of formula (I) according to claim 1 wherein X represents a methylene group.

5. Compound of formula (I) according to claim 1 which is {6-[(4-chlorophenylsulphonyl)amino]-2-methyl-5,6,7,8-tetrahydronaphth-1-yl}propionic acid, and also the addition salts thereof with a pharmaceutically acceptable base.

6. Process for the preparation of compounds of formula (I) according to claim 1 wherein X represents a methylene group, characterised in that there is used as starting material a sulphonyl chloride of formula (II) :
R₄-SO₂Cl (II)
wherein R₄ is as defined for formula (I),
which is reacted in basic medium :
either with a cyclohexylamine of formula (III) : 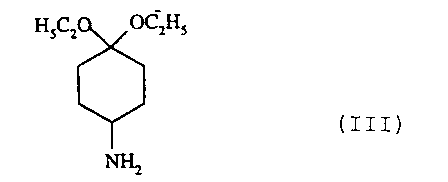 (prepared from the corresponding cyclohexanone by reaction, under an inert atmosphere, with benzylamine in the presence of sodium triacetoxyborohydride followed by hydrogenolysis)
to yield the compound of formula (IV) : 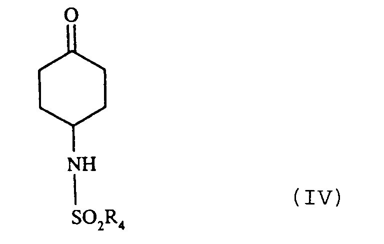 wherein R₄ is as defined for formula (I),
or with 4-aminocyclohexanol,
to yield the compound of formula (V) : 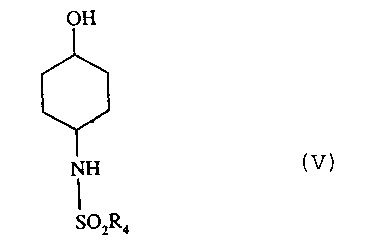 wherein R₄ is as defined for formula (I),
which is oxidised with the aid of Jones reagent (chromic acid in acetone and aqueous sulphuric acid),
to yield the compound of formula (IV) described above,
which compound of formula (IV) is reacted with ethyl formate in the presence of sodium hydride
to yield the compound of formula (VI) : 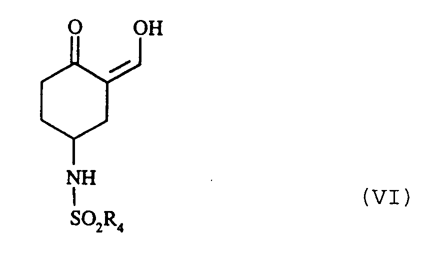 wherein R₄ is as defined for formula (I),
which is then subjected, depending on the nature of the compounds of formula (I) it is desired to obtain, to the action of an alkoxycarbonylmethylenetriphenylphosphorane optionally substituted in the α-position of the ester group by a halogen atom, a straight-chain or branched (C₁-C₆)-alkyl group, a phenyl group (unsubstituted or substituted by one or more halogen atoms or straight-chain or branched (C₁-C₆)-alkyl, straight-chain or branched (C₁-C₆)-alkoxy or trihalomethyl groups), a benzyl group, a pyridylmethyl group or by an imidazolylmethyl group,
to yield the compound of formula (VII) : 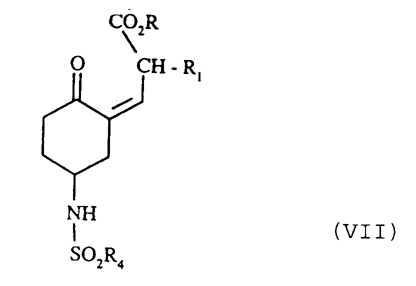 wherein R₁ and R₄ are as defined for formula (I) and R represents a straight-chain or branched (C₁-C₆)-alkyl group,
which is then subjected to reaction with p-toluene-sulphonic acid or trifluoroacetic acid
to yield the compound of formula (VIII) : 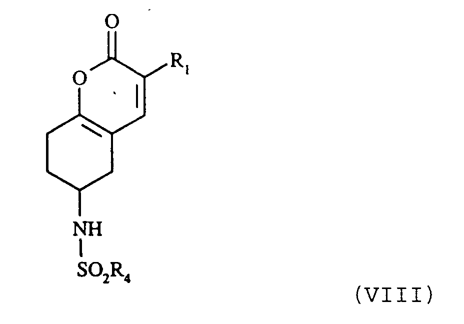 wherein R₁ and R₄ are as defined for formula (I),
which is subjected to a Diels-Alder reaction with a suitably substituted alkyl alk-2-ynoate
to yield, after possible rearrangement, the compound of formula (IX) : 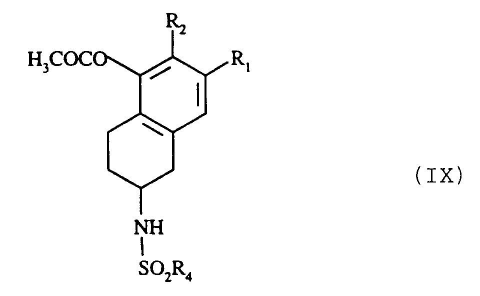 wherein R₁, R₂ and R₄ are as defined for formula (I),
which is subjected to the action of lithium aluminium hydride in anhydrous medium
to yield the compound of formula (X) : 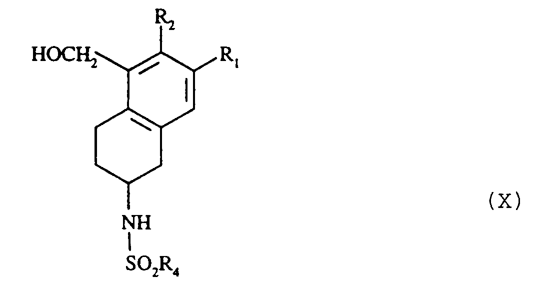 wherein R₁, R₂ and R₄ are as defined for formula (I),
which is reacted with an oxidizing agent, such as 4-benzylpyridinium dichromate,
to yield the aldehyde of formula (XI) : 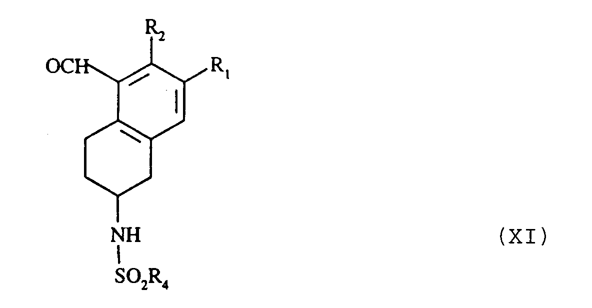 wherein R₁, R₂ and R₄ are as defined for formula (I),
which is reacted with (methoxycarbonylmethylene)triphenylphosphorane
to yield the compound of formula (XII): 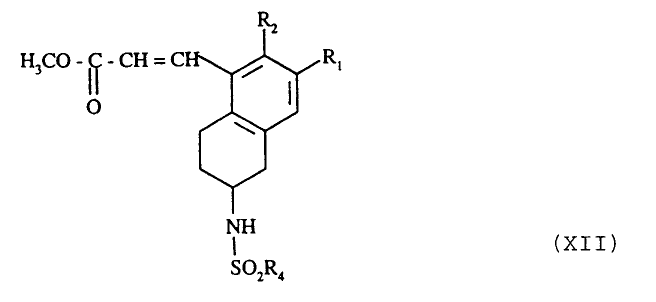 wherein R₁, R₂ and R₄ are as defined for formula (I),
which is reduced with the aid of samarium iodide in the presence of methanol
to yield the compound of formula (XIII) : 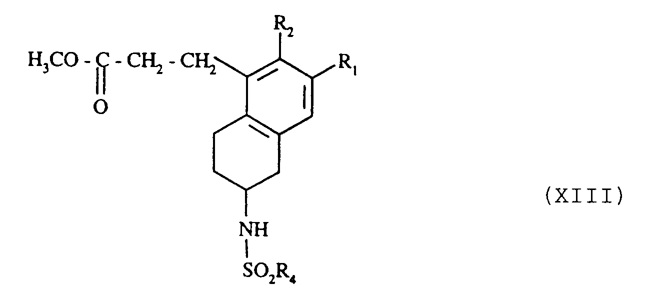 wherein R₁, R₂ and R₄ are as defined for formula (I),
which is then converted into the corresponding acid, ester or amide according to a conventional technique of organic chemistry,
to yield the compound of formula (I/a), a particular case of compounds of formula (I) : 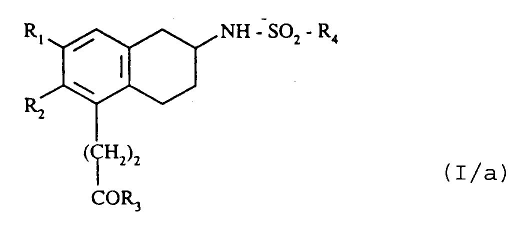 wherein R₁, R₂, R₃ and R₄ are as defined for formula (I),
- which may, where necessary, be purified according to a conventional purification technique,
- which is separated, where appropriate, into its isomers according to a conventional separation technique,
- which is converted, if desired, into its addition salts with a pharmaceutically acceptable base.

7. Process for the preparation of compounds of formula (I) according to claim 1 wherein R₁ and R₂ represent a hydrogen atom and X represents a methylene group,
characterised in that there is used as starting material a compound of formula (XIV) : 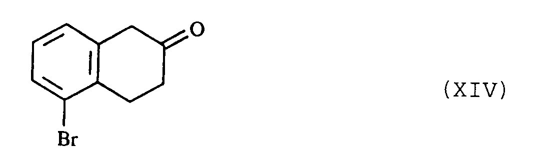 which is reacted with excess methyl acrylate in the presence of a catalytic amount of palladium acetate and tri-ortho-tolylphosphine in triethylamine,
to yield the compound of formula (XV) : 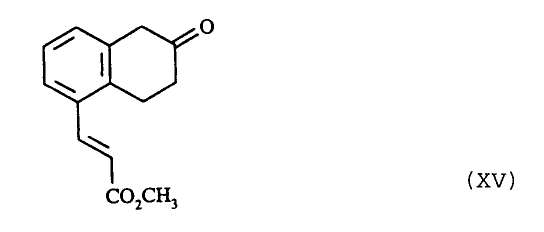 which is converted by reaction with benzylamine, in the presence of sodium triacetoxyborohydride, into the compound of formula (XVI) : 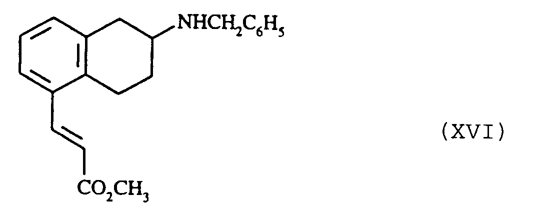 which is subjected to catalytic hydrogenation to yield the compound of formula (XVII) : 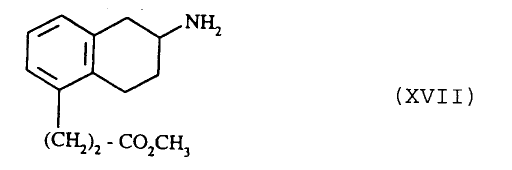 which is reacted in basic medium with a sulphonyl chloride of formula (II) :
R₄SO₂Cl (II)
wherein R₄ is as defined for formula (I),
to yield the compound of formula (XVIII) : 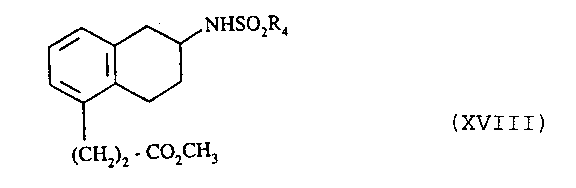 wherein R₄ is as defined for formula (I),
which is then converted into the corresponding acid, ester or amide according to a conventional technique of organic chemistry,
to yield the compound of formula (I/b), a particular case of compounds of formula (I) : 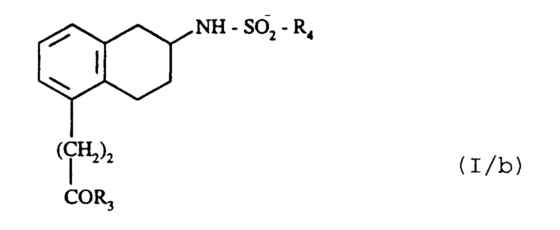 wherein R₃ and R₄ are as defined for formula (I),
- which may, where necessary, be purified according to a conventional purification technique,
- which is separated, where appropriate, into its isomers according to a conventional separation technique,
- which is converted, if desired, into its addition salts with a pharmaceutically acceptable base.

8. Process for the preparation of compounds of formula (I) according to claim 1 wherein X=X' represents a sulphur or oxygen atom, characterised in that there is used as starting material a compound of formula (XIX) : 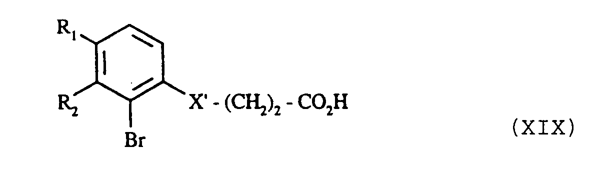 wherein R₁ and R₂ are as defined for formula (I) and X' represents a sulphur or oxygen atom,
which is cyclised in the presence of polyphosphoric acid
to yield the compound of formula (XX) : 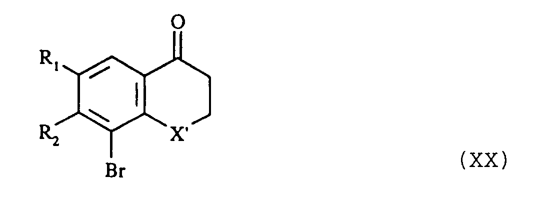 wherein R₁, R₂ and X' are as defined above,
which is reacted with excess methyl acrylate in the presence of a catalytic amount of palladium acetate and tri-ortho-tolylphosphine in triethylamine,
to yield the compound of formula (XXI) : 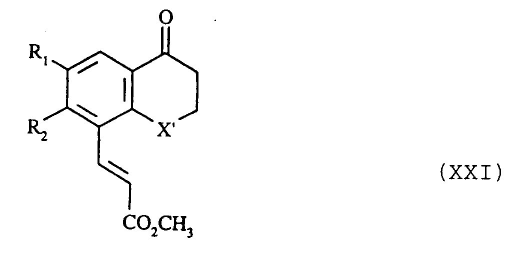 wherein R₁, R₂ and X' are as defined above,
which is reacted with hydroxylamine, then with tosyl chloride and finally is subjected to Neber rearrangement
to yield the compound of formula (XXII) : 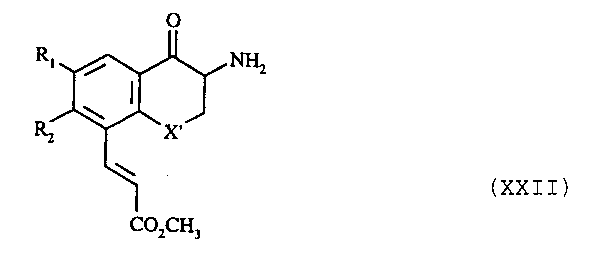 wherein R₁, R₂ and X' are as defined above,
which is then subjected to reduction in the presence of a catalyst to yield the compound of formula (XXIII) : 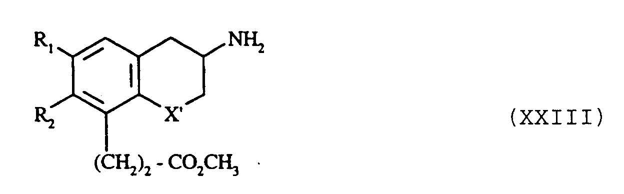 wherein R₁, R₂ and X' are as defined above,
which is reacted in basic medium with a sulphonyl chloride of formula (II) :
R₄SO₂Cl (II)
wherein R₄ is as defined for formula (I),
to yield the compound of formula (XXIV) : 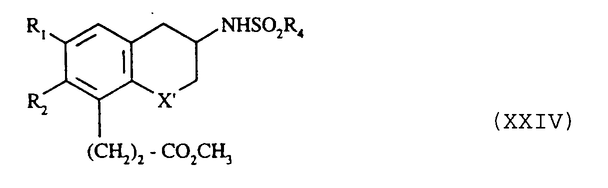 wherein R₁, R₂, R₄ and X' are as defined above,
which is then converted into the corresponding acid, ester or amide according to a conventional technique of organic chemistry,
to yield the compound of formula (I/c), a particular case of compounds of formula (I) : 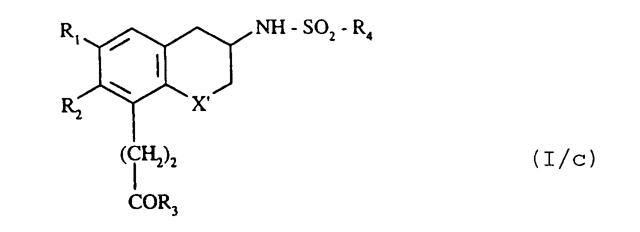 wherein R₁, R₂, R₃, R₄ and X' are as defined above,
- which may, where necessary, be purified according to a conventional purification technique,
- which is separated, where appropriate, into its isomers according to a conventional separation technique,
- which is converted, if desired, into its addition salts with a pharmaceutically acceptable base.

9. Process for the preparation of compounds of formula (I) according to claim 1 wherein X represents a methylene group, R₁ represents a methyl group and R₂ represents a hydrogen atom, characterised in that there is used as starting material 1,3-cyclohexanedione which is reacted with tiglic aldehyde to yield the compound of formula (XXV) : 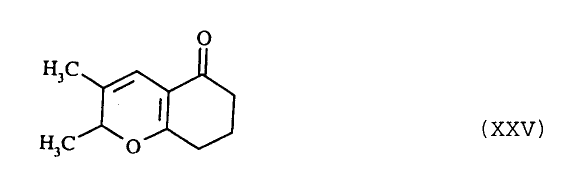 which is reacted with an alkyl propiolate to yield the compound of formula (XXVI) : 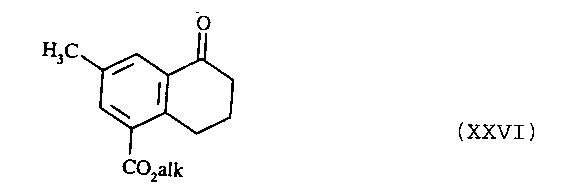 wherein alk represents an alkyl group, which is reacted with hydroxylamine, then with tosyl chloride and finally is subjected to Neber rearrangement
to yield the compound of formula (XXVII) : 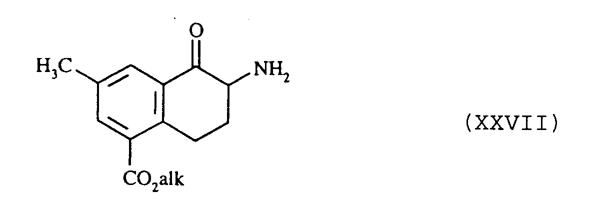 wherein alk is as defined above, which is then subjected to catalytic reduction, and subsequently to the action of a sulphonyl chloride of formula (II) according to claim 6,
to yield the compound of formula (XXVIII) : 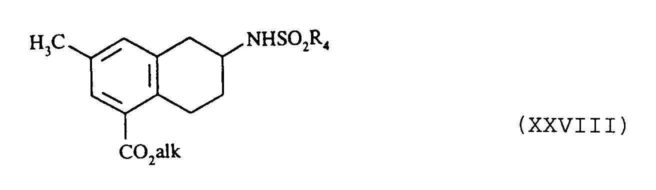 wherein R₄ and alk are as defined above,
which compound of formula (XXVIII) is then subjected to the sequence of reactions according to claim 6 described for the conversion of the compound of formula (IX) into the compound of formula (I/a), and yields the corresponding compound of formula (I).

10. Pharmaceutical compositions comprising as active ingredient at least one compound according to any one of claims 1 to 5, alone or in combination with one or more pharmaceutically acceptable, inert, non-toxic carriers.

11. Pharmaceutical composition according to claim 10, comprising at least one active ingredient according to any one of claims 1 to 5 for use as an antithrombotic agent.

## Patentansprüche

1. Verbindungen der Formel (I): 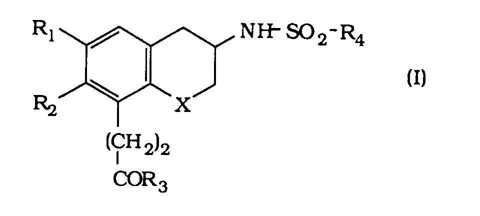 in der:
R₁ und R₂, die gleichartig oder verschieden sein können, jeweils ein Wasserstoffatom, ein Halogenatom, eine geradkettige oder Verzweigte (C₁-C₆)-Alkylgruppe, eine Phenylgruppe (die gegebenenfalls durch eines oder mehrere Halogenatome oder geradkettige oder verzweigte (C₁-C₆)-Alkylgruppen, geradkettige oder Verzweigte (C₁-C₆)-Alkoxygruppen oder Trihalogenomethylgruppen substituiert ist), eine Benzylgruppe, eine Pyridylmethylgruppe oder eine Imidazolylmethylgruppe, Thiazolylmethylgruppe, Pyridylgruppe, Imidazolylgruppe oder Thiazolylgruppe bedeuten,
oder
R₁ und R₂ gemeinsam mit den Kohlenstoffatomen, an die sie gebunden sind, einen Cyclopentan- oder Cyclohexan-ring bilden
R₃ eine Hydroxylgruppe, eine geradkettige oder verzweigte (C₁-C₆)-Alkoxygruppe oder eine Aminogruppe (die gegebenenfalls durch eine oder zwei geradkettige oder verzweigte (C₁-C₆)-Alkylgruppen substituiert ist), darstellt,
R₄ eine geradkettige oder verzweigte (C₁-C₆)-Alkylgruppe, eine Phenylgruppe (die gegebenenfalls durch ein oder mehrere Halogenatome, geradkettige oder verzweigte (C₁-C₆)-Alkylgruppen, geradkettige oder verzweigte (C₁-C₆)-Alkoxygruppen, Trihalogenomethylgruppen oder Hydroxylgruppen substituiert ist), eine Naphthylgruppe, eine Pyridylgruppe, eine Thienylgruppe oder eine Thiazolylgruppe darstellt und
X eine Methylengruppe, ein Sauerstoffatom oder ein Schwefelatom bedeutet,
deren Enantiomere sowie deren Additionssalze mit einer pharmazeutisch annehmbaren Base.

2. Verbindungen der Formel (I) nach Anspruch 1, worin R₁ ein Wasserstoffatom oder eine Methylgruppe darstellt.

3. Verbindungen der Formel (I) nach Anspruch 1, worin R₂ eine Methylgruppe bedeutet.

4. Verbindungen der Formel (I) nach Anspruch 1, worin X eine Methylengruppe darstellt.

5. Verbindungen der Formel (I) nach Anspruch 1, nämlich {6-[(4-Chlorphenylsulfonyl)-amino]-2-methyl-5,6,7,8-tetrahydronaphth-1-yl)-propionsäure sowie deren Additionssalze mit einer pharmazeutisch annehmbaren Base.

6. Verfahren zur Herstellung der Verbindungen der Formel (I) nach Anspruch 1, in der X eine Methylengruppe darstellt, **dadurch gekennzeichnet**, daß man als Ausgangsprodukt ein Sulfonylchlorid der Formel (II) einsetzt:
R₄ - SO₂Cl (II)
in der R₄ die bezuglich der Formel (I) angegebenen Bedeutungen besitzt, welches man in basischem Medium
entweder mit einem Cyclohexylamin der Formel (III) (welches man ausgehend von dem entsprechenden Cyclohexanon durch Reaktion mit Benzylamin in Gegenwart von Natriumtriacetoxyborhydrid unter einer inerten Atmosphäre und anschließende Hydrogenolyse hergestellt hat) umsetzt: 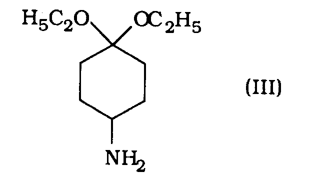 zur Bildung der Verbindung der Formel (IV): 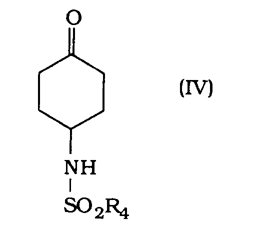 in der R₄ die gleiche Bedeutung besitzt wie in der Formel (I),
oder mit 4-Aminocyclohexanol umsetzt,
zur Bildung der Verbindung der Formel (V): 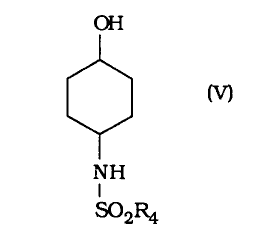 in der R₄ die gleiche Bedeutung besitzt wie in der Formel (I),
welche man mit Hilfe des Jones-Reagens (Chromsäure in Aceton und wäßriger Schwefelsäure) oxidiert,
zur Bildung der oben beschriebenen Verbindung der Formel (IV), welche Verbindung der Formel (IV)
man mit Ameisensäureethylester in Gegenwart von Natriumhydrid umsetzt zur Bildung der Verbindung der Formel (VI): 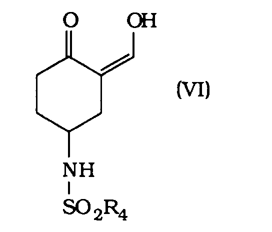 in der R₄ die gleiche Bedeutung besitzt wie in der Formel (I),
welche man anschließend in Abhängigkeit von der Art der herzustellenden Verbindungen der Formel (I) der Einwirkung eines Carbalkoxymethylen-triphenylphosphorans, das gegebenenfalls in der α-Stellung der Estergruppe durch ein Halogenatom, eine geradkettige oder verzweigte (C₁-C₆)-Alkylgruppe, eine Phenylgruppe (die gegebenenfalls durch ein oder mehrere Halogenatome, geradkettige oder verzweigte (C₁-C₆)-Alkylgruppen, geradkettige oder verzweigte (C₁-C₆)-Alkoxygruppen oder Trihalogenomethylgruppen substituiert ist), eine Benzylgruppe, eine Pyridylmethylgruppe oder eine Imidazolylmethylgruppe substituiert ist, unterwirft,
zur Bildung der Verbindung der Formel (VII): 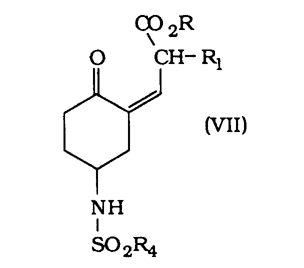 in der R₁ und R₄ die gleichen Bedeutungen besitzen wie in der Formel (I) und Reine geradkettige oder verzweigte (C₁-C₆)-Alkylgruppe darstellt,
welche man anschließend mit p-Toluolsulfonsäure oder Trifluoressigsäure umsetzt
zur Bildung der Verbindung der Formel (VIII): 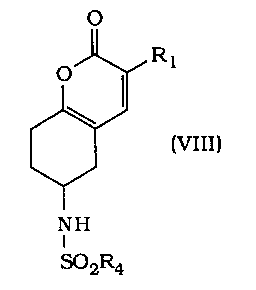 in der R₁ und R₄ die gleichen Bedeutungen besitzen wie in der Formel (I),
welche man einer Diels-Alder-Reaktion mit einem in geeigneter Weise substituierten Alk-2-in-säurealkylester unterzieht,
so daß man nach eventuellen Umwandlungen die Verbindung der Formel (IX) erhält: 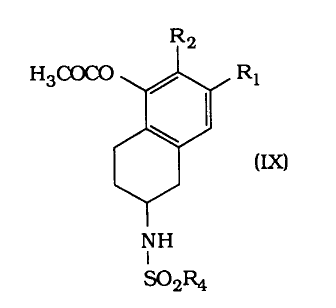 in der R₁, R₂ und R₄ die gleiche Bedeutung besitzen wie in der Formel (I),
welche man der Einwirkung von Lithiumaluminiumhydrid in wasserfreiem Medium unterwirft
zur Bildung der Verbindung der Formel (X): 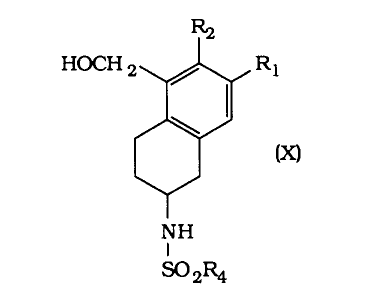 in der R₁, R₂ und R₄ die gleiche Bedeutung besitzen wie in der Formel (I),
welche man mit einem Oxidationsmittel, wie 4-Benzylpyridinium-dichromat umsetzt
zur Bildung des Aldehyds der Formel (XI): 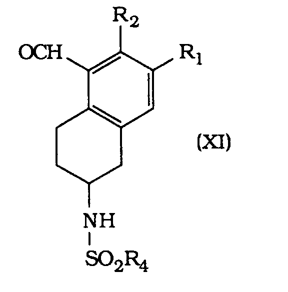 in der R₁, R₂ und R₄ die gleiche Bedeutung besitzen wie in der Formel (I),
welchen man mit (Carbomethoxymethylen)-triphenylphosphoran umsetzt
zur Bildung der Verbindung der Formel (XII): 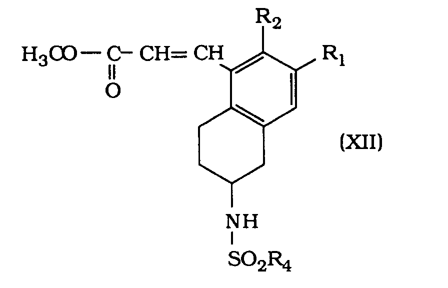 in der R₁, R₂ und R₄ die gleiche Bedeutung besitzen wie in der Formel (I),
welche man mit Samariumiodid in Gegenwart von Methanol reduziert
zur Bildung der Verbindung der Formel (XIII): 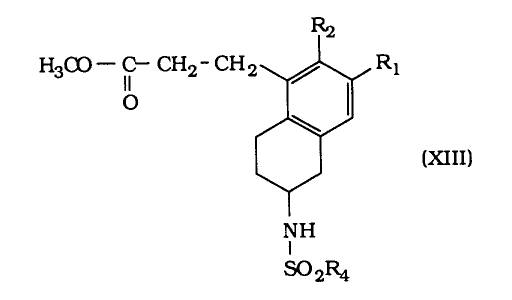 in der R₁, R₂ und R₄ die gleiche Bedeutung besitzen wie in der Formel (I),
welche man anschließend mit Hilfe einer klassischen Methode der organischen Chemie in die entsprechende Säure, den entsprechenden Ester oder das entsprechende Amid umwandelt
zur Bildung der Verbindung der Formel (I/a), einem Sonderfall der Verbindungen der Formel (I): 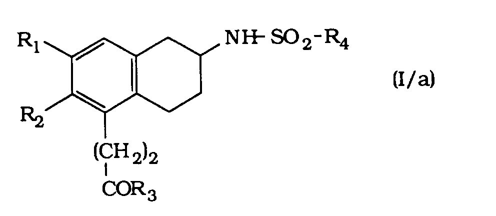 in der R₁, R₂, R₃ und R₄ die gleiche Bedeutung besitzen wie in der Formel (I),
- welche man gegebenenfalls mit Hilfe einer klassischen Reinigungsmethode reinigt,
- welche man gegebenenfalls mit Hilfe einer klassischen Trennmethode in die Isomeren auftrennt oder
- welche man gewünschtenfalls in die Additionssalze mit einer pharmazeutisch annehmbaren Base überführt.

7. Verfahren zur Herstellung der Verbindungen der Formel (I) nach Anspruch 1, in der R₁ und R₂ ein Wasserstoffatom und X eine Methylengruppe bedeuten, **dadurch gekennzeichnet**, daß man als Ausgangsprodukt eine Verbindung der Formel (XIV) einsetzt: 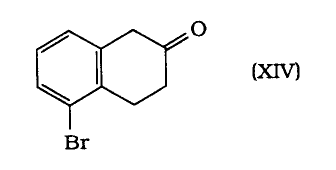 welche man in Gegenwart einer katalytischen Menge Palladiumacetat und von Triorthotolylphosphin in Triethylamin mit einem Überschuß von Acrylsäuremethylester umsetzt
zur Bildung der Verbindung der Formel (XV): 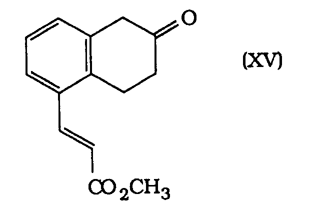 welche man durch Reaktion mit Benzylamin in Gegenwart von Natriumtriacetoxyborhydrid in die Verbindung der Formel (XVI) umwandelt: 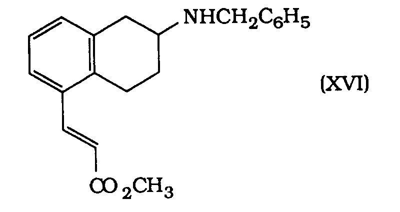 welche man einer katalytischen Hydrierung unterwirft zur Bildung der Verbindung der Formel (XVII): 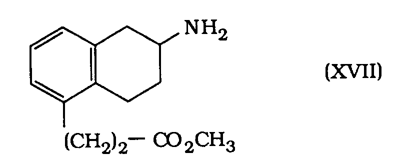 welche man mit Sulfonylchlorid der Formel (II) in basischem Medium umsetzt:
R₄SO₂Cl (II)
in der R₄ die gleiche Bedeutung besitzt wie in der Formel (I),
zur Bildung der Verbindung der Formel (XVIII): 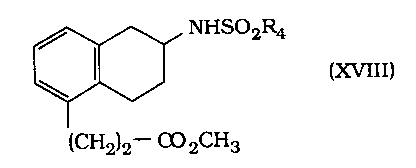 in der R₄ die gleiche Bedeutung besitzt wie in der Formel (I),
welche man anschließend mit Hilfe einer klassischen Methode der organischen Chemie in die entsprechende Säure, den entsprechenden Ester oder das entsprechende Amid umwandelt,
zur Bildung der Verbindung der Formel (I/b), einem Sonderfall der Verbindungen der Formel (I): 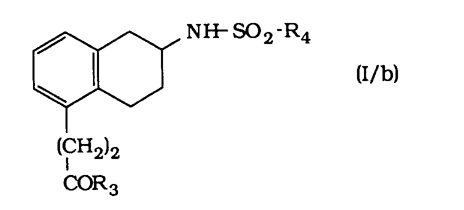 in der R₃ und R₄ die gleiche Bedeutung besitzen wie in der Formel (I),
- welche man gegebenenfalls mit Hilfe einer klassischen Reinigungsmethode reinigt,
- welche man gegebenenfalls mit Hilfe einer klassischen Trennmethode in die Isomeren auftrennt oder
- welche man gewünschtenfalls in die Additionssalze mit einer pharmazeutisch annehmbaren Base überführt.

8. Verfahren zur Herstellung der Verbindungen der Formel (I) nach Anspruch 1, in der X = X' ein Schwefelatom oder ein Sauerstoffatom bedeutet, **dadurch gekennzeichnet**, daß man als Ausgangsprodukt eine Verbindung der Formel (XIX) einsetzt: 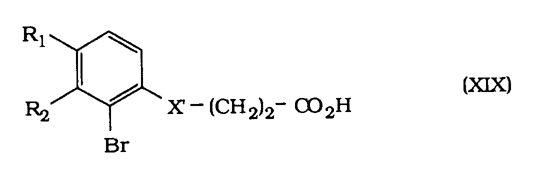 in der R₁ und R₂ die gleiche Bedeutung besitzen wie in der Formel (I) und X' ein Schwefelatom oder ein Sauerstoffatom darstellt,
welche man in Gegenwart von Polyphosphorsäure cyclisiert zur Bildung der Verbindung der Formel (XX): 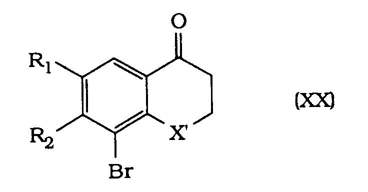 in der R₁, R₂ und X' die gleiche Bedeutung besitzen wie oben angegeben, welche man in Gegenwart einer katalytischen Menge Palladiumacetat und von Triorthotolylphosphin in Triethylamin mit einem Überschuß von Acrylsäuremethylester umsetzt
zur Bildung der Verbindung der Formel (XXI): 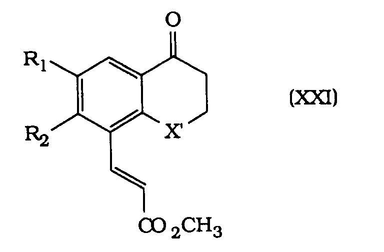 in der R₁, R₂ und X' die gleiche Bedeutung besitzen wie oben angegeben,
welche man mit Hydroxylamin und dann mit Tosylchlorid umsetzt und schließlich einer Néber-Umwandlung unterzieht zur Bildung der Verbindung der Formel (XXII): 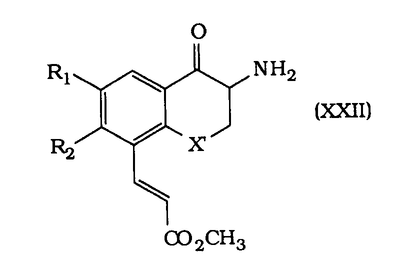 in der R₁, R₂ und X' die gleiche Bedeutung besitzen wie oben angegeben,
welche man dann in Gegenwart eines Katalysators reduziert zur Bildung der Verbindung der Formel (XXIII): 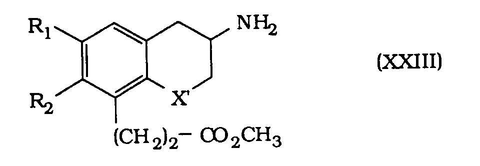 in der R₁, R₂ und X' die gleiche Bedeutung besitzen wie oben angegeben,
welche man mit einem Sulfonylchlorid der Formel (II) in basischem Medium umsetzt:
R₄SO₂Cl (II)
in der R₄ die gleiche Bedeutung besitzt wie in der Formel (I),
zur Bildung der Verbindung der Formel (XXIV): 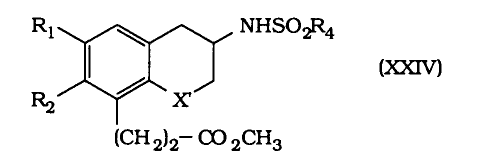 in der R₁, R₂, R₄ und X' die gleiche Bedeutung besitzen wie oben angegeben,
welche man anschließend mit Hilfe einer klassischen Methode der organischen Chemie in die entsprechende Säure, den entsprechenden Ester oder das entsprechende Amid umwandelt,
zur Bildung der Verbindung der Formel (I/c), einem Sonderfall der Verbindungen der Formel (I): 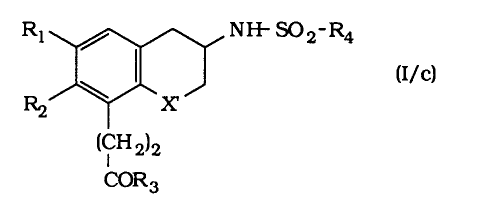 in der R₁, R₂, R₃, R₄ und X' die gleiche Bedeutung besitzen wie oben angegeben,
- welche man gegebenenfalls mit Hilfe einer klassischen Reinigungsmethode reinigt,
- welche man gegebenenfalls mit Hilfe einer klassischen Trennmethode in die Isomeren auftrennt oder
- welche man gewünschtenfalls in die Additionssalze mit einer pharmazeutisch annehmbaren Base überführt.

9. Verfahren zur Herstellung der Verbindungen der Formel (I) nach Anspruch 1, in der X eine Methylengruppe, R₁ eine Methylgruppe und R₂ ein Wasserstoffatom bedeuten, **dadurch gekennzeichnet**, daß man als Ausgangsprodukt 1,3-Cyclohexandion verwendet, welches man mit Tiglinaldehyd umsetzt zur Bildung der Verbindung der Formel (XXV): 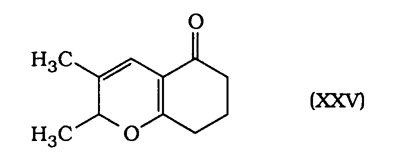 welche man mit Propiolsäurealkylester umsetzt zur Bildung der Verbindung der Formel (XXVI): 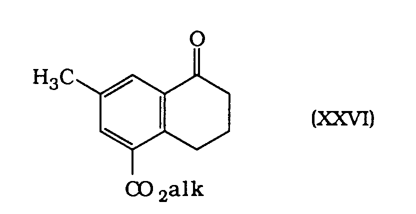 in der alk eine Alkylgruppe darstellt, welche man mit Hydroxylamin und dann mit Tosylchlorid umsetzt und anschließend einer Neber-Umwandlung unterwirft,
zur Bildung der Verbindung der Formel (XXVII): 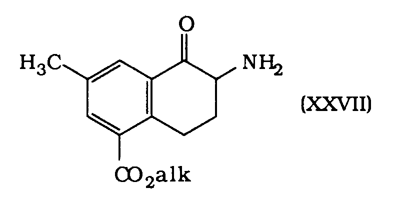 in der alk die gleiche Bedeutung besitzt wie oben angegeben, welche man dann einer katalytischen Reduktion und dann der Einwirkung des Sulfonylchlorids der Formel (II) gemäß Anspruch 6 unterwirft,
zur Bildung der Verbindung der Formel (XXVIII): 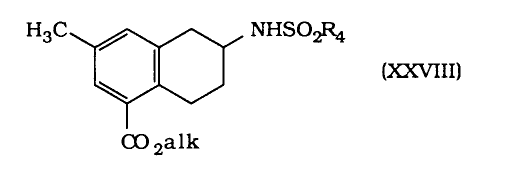 in der R₄ und alk die gleiche Bedeutung besitzen wie oben angegeben,
welche Verbindung der Formel (XVIII) man anschließend den Reaktionen gemäß Anspruch 6 bezüglich der Umwandlung der Verbindung der Formel (IX) in die Verbindung der Formel (I/a) unterwirft, was zu der entsprechenden Verbindung der Formel (I) führt.

10. Pharmazeutische Zubereitungen enthaltend als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien.

11. Pharmazeutische Zubereitung nach Anspruch 10 enthaltend mindestens einen Wirkstoff gemäß einem der Ansprüche 1 bis 5 als Antithrombotikum.
